# EUROPEAN PATENT APPLICATION

(11) **EP 4 793 287 A1**
(43) Date of publication of application: **19.08.2026**
(21) Application number: 24876680.0
(22) Date of filing: 12.10.2024
(51) Int. Cl.: C07K 16/28, C12N 15/13, A61K 39/395, A61K 47/68, A61P 35/00, A61P 35/02, A61P 37/02, G01N 33/68

(54) **TUMOR MICROENVIRONMENT SPECIFICALLY ACTIVATED CD47 ANTIBODY**

(30) Priority: 13.10.2023 CN 202311328177
(71) Applicant: Affinity (Shanghai) Biopharmaceutical Co., Ltd., Shanghai 201203 (CN)
(72) Inventor: DING, Chengli, Guangzhou, Guangdong 510700 (CN); LIU, Yuan, Guangzhou, Guangdong 510700 (CN); LIU, Chen, Guangzhou, Guangdong 510700 (CN)
(74) Representative: Mewburn Ellis LLP
(86) International application number: PCT/CN2024/124413
(87) International publication number: WO 2025/077875

(57) **Abstract**

Provided is a tumor microenvironment specifically activated CD47 antibody. Specifically, provided is a bispecific antibody which is, in a chemical coupling manner, linked to a substrate and a blocker which are highly expressed in a tumor environment and can recognize cleaving. The antibody activity of the bispecific antibody in a normal tissue is suppressed, and the antibody activity of the bispecific antibody is activated in a tumor environment. In addition, the bispecific antibody also has a significantly reduced toxic and side effect.

## Description

### TECHNICAL FIELD

The present invention relates to the field of antibodies, and in particular to a tumor microenvironment specifically activated CD47 antibody.

### BACKGROUND

CD47 is a membrane protein involved in the increase in intracellular calcium concentration during cell adhesion to the extracellular matrix. The encoded protein is also a receptor for the C-terminal cell-binding domain of coagulase and may play a role in membrane trafficking and signal transduction. The CD47-SIRPα (signal regulatory protein α) pathway, also known as the "don't eat me" signal, was first discovered in 1990. SIRPα is the first protein member of the SIRP family to be discovered and is present in myeloid cells and includes all forms of macrophages.

SIRPα has an extracellular immunoglobulin functional region that binds to the ligand and an intracellular inhibitory motif containing ITIM (immunoreceptor tyrosine-based inhibitory motif), which can bind to SHP-1 (sh2 motif-containing Protein Tyrosine Phosphatase 1) or SHP2 (also known as PTPN11) to transmit information.

Ovarian cancer tumor heterogeneity and the surrounding tumor microenvironment (TME) have been reported to influence the efficacy of immunotherapy and patient prognosis. The TME of ovarian cancer is inherently heterogeneous. CD47 plays an important role in cell functional behavior and immune homeostasis related to cancer prognosis.

CD47 has a long N-terminal extracellular domain, five transmembrane domains, and a short intracellular domain. CD47 binds to SIRPα and induces activation of SHP1 and SHP2 by tyrosine phosphorylation of the SIRPαITIM, leading to dephosphorylation of myosin IIA and inhibition of cytoskeletal reorganization, which is a step required for macrophage phagocytosis of target cells.

The role of CD47 in phagocytosis was discovered in CD47-deficient mice. If red blood cells were removed from CD47-deficient mice and given to wild-type mice, the CD47-deficient red blood cells were quickly cleared, while inhibiting macrophages reversed this effect, indicating that macrophages play a crucial role in phagocytosis.

In addition to regulating normal tissue homeostasis, CD47 has also been found to be abundantly expressed on tumor cells (both hematological and non-hematological cells). CD47 is not directly involved in regulating tumor cell proliferation and survival. Antibody inhibition of CD47 can increase tumor cell phagocytosis even in the absence of T cells, B cells, and NK cells, suggesting that tumor cells can evade macrophage phagocytosis and thus avoid clearance by macrophages.

The anti-CD47 antibody developed in the present invention has low toxicity and high safety to human body.

### SUMMARY OF THE INVENTION

The purpose of the present invention is to provide a tumor microenvironment specifically activated CD47 antibody.

In the first aspect of the present invention, it provides a mutant CD47 antibody, wherein the mutant CD47 antibody has one or more mutations introduced into the complementarity determining regions (CDRs) of the heavy chain variable region and/or the light chain variable region relative to the sequences of wild-type CD47 antibody, and the mutations are selected from the group consisting of:
substitution of one or more amino acids with cysteine (Cys) at positions Y33, R55, Y61, W65, Y105, or L107 of the heavy chain variable region, and/or at positions Q27, Y49, T53, F92, Y93, Y96, or N98 of the light chain variable region;
wherein, the binding affinity of the wild-type CD47 antibody for CD47 is E0, the binding affinity of the mutant CD47 antibody for CD47 is E1, and E1/E0 ≤ 5.5 (preferably E1/EO ≤ 3, preferably ≤ 1.5, more preferably E1/E0 ≤ 1);
wherein, the wild-type CD47 antibody comprises a heavy chain variable region and a light chain variable region, and the heavy chain variable region comprises the following three heavy chain variable regions:
   HCDR1 SGYDMC (SEQ ID NO. 18),
   HCDR2 CIYTRSSGGTYYASWAKG (SEQ ID NO. 19),
   HCDR3 GRSSYRLNL (SEQ ID NO. 20), and
the light chain variable region comprises the following three light chain variable regions:
   LCDR1 QASQSISNELS (SEQ ID NO. 21),
   LCDR2 RASTLES (SEQ ID NO. 22),
   LCDR3 QSTFYGDYGNA (SEQ ID NO. 23);
wherein, any of the above amino acid sequences further comprises a derivative sequence optionally subjected to addition, deletion, modification and/or substitution of at least one amino acid, and capable of retaining CD47 binding affinity.

In another preferred embodiment, the amino acid sequence of any of the above CDRs contains a derivative CDR sequence subjected to addition, deletion, modification and/or substitution of 1, 2 or 3 amino acids, and the derivative antibody composed of VH and VL containing the derived CDR sequence can retain CD47 binding affinity.

In another preferred embodiment, the number of amino acids subjected to addition, deletion, modification and/or substitution is 1-5 (such as 1-3, preferably 1-2, more preferably 1).

In another preferred embodiment, the derivative sequence subjected to addition, deletion, modification and/or substitution of at least one amino acid and capable of retaining CD47 binding affinity is an amino acid sequence with a homology or sequence identity of at least 85%.

In another preferred embodiment, the sequence identity is of 86%, 87%, 88%, 89%, 90%, 91%, 92%, 93%, 94%, 95%, 96%, 97%, 98%, 99% or 100%.

In another preferred embodiment, the wild-type CD47 antibody includes: a single-chain antibody, a double-chain antibody, a monoclonal antibody, a chimeric antibody (such as a human-murine chimeric antibody), a murine antibody, or a humanized antibody.

In another preferred embodiment, the heavy chain variable region of the mutant CD47 antibody comprises the following three heavy chain variable regions:
HCDR1 SGYDMC (SEQ ID NO. 18),
HCDR2 CIYTRSSGGTYCASWAKG (SEQ ID NO.24),
HCDR3 GRSSYRLNL (SEQ ID NO. 20), and

the light chain variable region comprises the following three light chain variable regions:
   LCDR1 QASQSISNELS (SEQ ID NO.21),
   LCDR2 RASTLES (SEQ ID NO.22),
   LCDR3 QSTFYGDYGNA (SEQ ID NO.23);
wherein, any of the above amino acid sequences further comprises a derivative sequence optionally subjected to addition, deletion, modification and/or substitution of at least one amino acid, and capable of retaining CD47 binding affinity.

In another preferred embodiment, the mutant CD47 antibody includes a PEGylated CD47 antibody.

In another preferred embodiment, the PEGylation is carried out using PEG with a molecular weight of 5-100 kD (preferably 10-50 kD).

In another preferred embodiment, the PEGylation includes site-specific PEGylation or non-site-specific PEGylation.

In another preferred embodiment, the CD47 binding affinity is represented by the EC50 value for binding to CD47.

In another preferred embodiment, the ratio (E1/E0) of the binding affinity of the mutant CD47 antibody for CD47 (E1) to the binding affinity of the corresponding wild-type CD47 antibody for CD47 (E0) is 0.4-5.5, preferably 0.5-2, preferably 0.7-1.5, and more preferably 0.8-1.2.

In another preferred embodiment, the wild-type CD47 antibody is a murine CD47 antibody or a humanized CD47 antibody.

In another preferred embodiment, the wild-type CD47 antibody comprises a heavy chain variable region as set forth in SEQ ID NO.7, and a light chain variable region as set forth in SEQ ID NO.8.

In another preferred embodiment, the amino acid sequence of the heavy chain variable region of the wild-type CD47 antibody has at least 80%, 85%, 86%, 87%, 88%, 89%, 90%, 91%, 92%, 93%, 94%, 95%, 96%, 97%, 98%, 99% or 100% sequence identity with the amino acid sequence of the heavy chain variable region of SEQ ID NO.7.

In another preferred embodiment, the amino acid sequence of the light chain variable region of the wild-type CD47 antibody has at least 80%, 85%, 86%, 87%, 88%, 89%, 90%, 91%, 92%, 93%, 94%, 95%, 96%, 97%, 98%, 99% or 100% sequence identity with the amino acid sequence of the light chain variable region of SEQ ID NO.8.

In another preferred embodiment, the humanized CD47 antibody comprises a heavy chain variable region as set forth in SEQ ID NO. 11, 12, 13 or 14, and a light chain variable region as set forth in SEQ ID NO. 15, 16 or 17; or comprises
an amino acid sequence of a heavy chain variable region that has at least 80%, 85%, 86%, 87%, 88%, 89%, 90%, 91%, 92%, 93%, 94%, 95%, 96%, 97%, 98%, 99% or 100% sequence identity with the sequence set forth in SEQ ID NO. 11, 12, 13 or 14; and
an amino acid sequence of a light chain variable region that has at least 80%, 85%, 86%, 87%, 88%, 89%, 90%, 91%, 92%, 93%, 94%, 95%, 96%, 97%, 98%, 99% or 100% sequence identity with the sequence set forth in SEQ ID NO. 15, 16 or 17.

In another preferred embodiment, the mutant CD47 antibody comprises one or more mutations (preferably 1, 2, 3, 4, or 5 amino acid mutations) in the complementarity determining regions (CDRs) of the heavy chain variable region and/or the light chain variable region relative to that of the wild-type CD47 antibody.

In another preferred embodiment, the mutant CD47 antibody comprises substitution of one or more amino acids with cysteine (Cys) in the complementarity determining regions (CDRs) of the heavy chain variable region relative to that of the wild-type CD47 antibody; and/or comprises substitution of one or more amino acids with cysteine (Cys) in the complementarity determining regions (CDRs) of the light chain variable region relative to that of the wild-type CD47 antibody.

In another preferred embodiment, the mutant CD47 antibody comprises a mutation in the complementarity determining regions corresponding to the heavy chain variable region of the wild-type CD47 antibody, wherein the mutation is selected from the group consisting of: Y33C, R55C, Y61C, W65C, Y105C, L107C, and a combination thereof, preferably a Y61C mutation; and/or
a mutation in the complementarity determining regions corresponding to the light chain variable region of the wild-type CD47 antibody, wherein the mutation is selected from the group consisting of: Q27C, Y49C, T53C, F92C, Y93C, Y96C, N98C, and a combination thereof, preferably a Y49C mutation.

In another preferred embodiment, all position numbers for amino acids mentioned with respect to antibody amino acid residues are based on Kabat numbering.

In another preferred embodiment, the amino acid sequence of the heavy chain variable region of the mutant CD47 antibody has at least 80%, 85%, 86%, 87%, 88%, 89%, 90%, 91%, 92%, 93%, 94%, 95%, 96%, 97%, 98%, 99%, or 100% sequence identity with the amino acid sequence of the heavy chain variable region of SEQ ID NO. 7, 11, 12, 13, or 14; and
the amino acid sequence of the light chain variable region of the mutant CD47 antibody has at least 80%, 85%, 86%, 87%, 88%, 89%, 90%, 91%, 92%, 93%, 94%, 95%, 96%, 97%, 98%, 99%, or 100% sequence identity with the amino acid sequence of the light chain variable region of SEQ ID NO. 8, 15, 16, or 17.

In another preferred embodiment, no interchain or intrachain disulfide bonds are formed within the complementarity determining regions (CDRs) of the variable regions of the antibody.

In another preferred embodiment, the mutant CD47 antibody comprises: (i) a Fab fragment; (ii) a F(ab')2 fragment; (iii) a Fd fragment; (iv) a Fv fragment; (v) a single-chain Fv (scFv) molecule; or (vi) a dAb fragment.

In another preferred embodiment, the mutant CD47 antibody is a bispecific, trispecific or multispecific antibody.

In another preferred embodiment, the mutant CD47 antibody includes a modified mutant CD47 antibody.

In another preferred embodiment, the modified mutant CD47 antibody is a tumor microenvironment-specific activated antibody (TMEAbody), that is, a masked antibody.

In another preferred embodiment, the modified mutant CD47 antibody includes, but is not limited to, linking a blocker to the cysteine at the mutation site of the mutant CD47 antibody via a cleavable linker peptide, wherein the mutation site is located in the complementarity determining regions (CDR regions) of the heavy chain variable region and/or the light chain variable region of the mutant CD47 antibody.

In another preferred embodiment, the mutant CD47 antibody includes a modified mutant CD47 antibody having the following structure:

Ab-(P-R1)n

wherein,
Ab is a mutant CD47 antibody;
R1 is each independently a blocker;
P is a cleavable linker peptide;
n is a positive integer of 1 to 5;
"-" represents a chemical bond or a linker or a connector.

In another preferred embodiment, the blocker refers to a moiety that prevents Ab from binding to its ligand or receptor.

In another preferred embodiment, the blocker is selected from groups containing a polyethylene glycol-C₁₋₅ -alkylcarbonyl group: 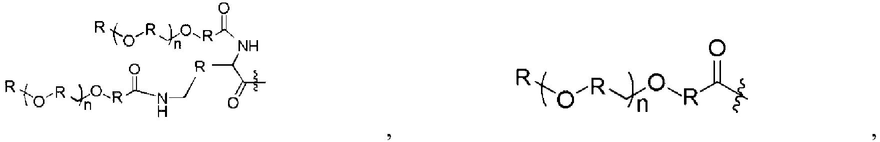 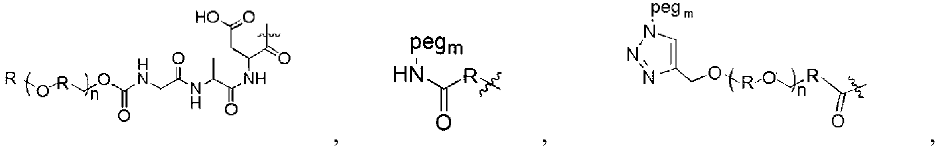 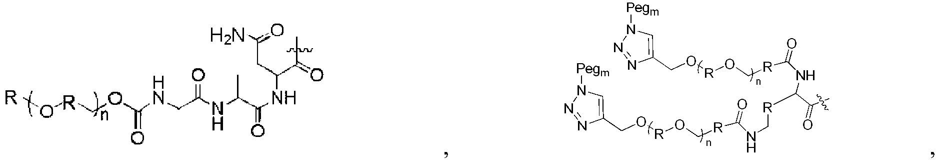 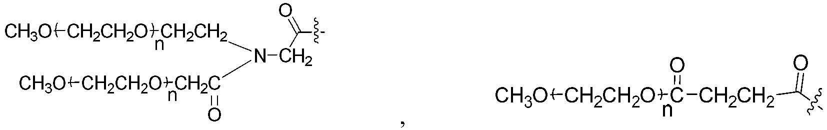 and 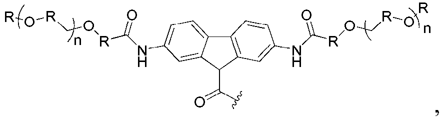 wherein each R is independently a C1-4 alkyl group; each n is independently an integer in the range of 1 to 30,000, such as an integer of 1 to 15000, 1 to 5000, 1 to 2000, 1 to 300, 1 to 150, 1 to 50, 1 to 20 or 3 to 12; polyethylene glycol or PEGₘ represents polyethylene glycol with a molecular weight of 44 to 132000, such as polyethylene glycol with a molecular weight of 1000 to 50000 or 10000 to 30000; m represents the molecular weight of the polyethylene glycol; the wavy line indicates the position connecting R2.

In another preferred embodiment, the blocker is selected from the group consisting of: PEG5K, PEG10K, PEG20K, and PEG40K.

In another preferred embodiment, the R1 is 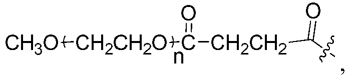 wherein n is independently an integer of 1 to 2000, 1 to 300, 1 to 150, 1 to 50 or 1 to 20.

In another preferred embodiment, the P comprises a structure as shown below:

R2-R3-R4,

wherein,
R2 is absent, or a cleavable linker arm that can be activated by one or more proteolytic enzymes, or a chemical bond that can be activated by acid in a pathological microenvironment;
R3 is absent, or a linker arm that automatically detaches after R2 is cleaved or activated by acid in a pathological microenvironment; when R2 is absent, R3 is a chemical bond that is activated by acid in a pathological microenvironment;
R4 is a group capable of covalently binding to the sulfur atom in the cysteine residue comprised in the mutant CD47 antibody.

In another preferred embodiment, the R1 is connected to R2 in P via a chemical bond.

In another preferred embodiment, the R2 is a peptide that can be activated or cleaved by one or more proteolytic enzymes, proteases or peptidases, wherein the protease is selected from the group consisting of asparagine endopeptidase, granzyme, cathepsin B, cathepsin C, cathepsin D, cathepsin E, cathepsin K, cathepsin L, kallikrein, hK1, hK10, hK15, plasmin, collagenase, type IV collagenase, astrin, factor Xa, chymotrypsin-like protease, trypsin-like protease, elastase-like protease, subtilisin-like protease, actinidin, bromelain, calpain, caspase, caspase-3, Mirl-CP, papain, HIV-1 protease, HSV protease, CMV protease, chymosin, peptidase, interstitial protease, plasma protease, pitcher plant protease, metalloexopeptidase, metalloendopeptidase, matrix metalloproteinase (MMP), MMP1, MMP2, MMP3, MMP8, MMP9, MMP10, MMP11, MMP12, MMP13, MMP14, ADAM10, ADAM12, urokinase plasminogen activator (uPA), enterokinase, prostate-specific antigen (PSA, hK3), interleukin-113 converting enzyme, thrombin, FAP (FAP-a), transmembrane peptidase (meprin), dipeptidyl peptidase and dipeptidyl peptidase-4 (DPPIV/CD26), preferably asparagine endopeptidase.

In another preferred embodiment, the R2 is a linker peptide that can be cleaved under the acidic conditions of the pathological microenvironment; preferably, R2 is a tripeptide, wherein the first amino acid residue of the tripeptide connected to R1 is selected from Ala, Thr, Val and Ile, the second amino acid residue in the middle is selected from Ala, Thr, Val and Asn, and the acid residue of the third amino acid connected to Ab is selected from Asn and Asp; wherein R2 is connected to R1 through the amino group of the first amino acid residue in the form of an amide, ester, carbamate, urea or hydrazone bond, and is connected to R3 through the carboxyl group of the third amino acid residue in the form of an amide, ester, carbamate, urea or hydrazone bond; preferably, the tripeptide is selected from Ala-Ala-Asn and Ala-Ala-Asp.

In another preferred embodiment, Ala-Ala-Asn can be recognized and cleaved by asparagine endopeptidase, and Ala-Ala-Asp can be recognized and cleaved by granzyme.

In another preferred embodiment, R2 is a linker peptide that can be cleaved under the acidic conditions of the pathological microenvironment and is selected from amide, ester, carbamate, urea and hydrazone bonds; preferably, the structure of R1-R2-R3-R4 is represented by the following formulas: 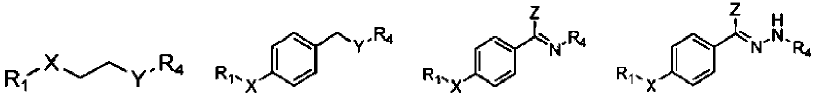 wherein X and Y are each independently NR' or O, Z is H or C1-10 alkyl, and R' is H or C1-4 alkyl; R3 is connected to R1 and R4 via X and Y, respectively, in the form of an amide, ester, urethane, urea, or hydrazone bond.

In another preferred embodiment, R4 is

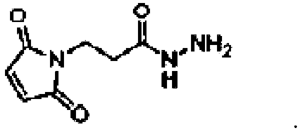

In another preferred embodiment, the structure of the P-R1 is as shown below, 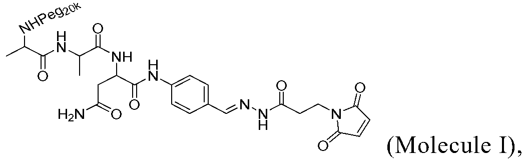 wherein R1 is PEG20K, and the structural formula is:

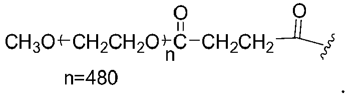

In another preferred embodiment, the structure of the P-R1 is as shown below, 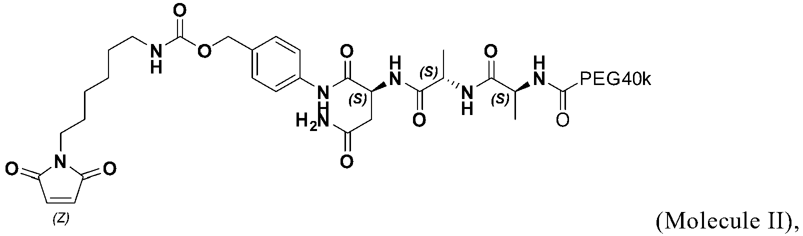 wherein R1 is PEG40K, and the structural formula is:

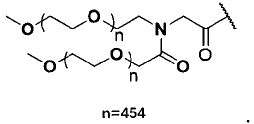

In another preferred embodiment, the modified mutant CD47 antibody is an activatable antibody.

In another preferred embodiment, the protease is produced by a tumor adjacent to cells expressing CD47 in the tissue and/or is produced by a tumor co-localized with CD47 in the tissue, and wherein when the activatable antibody is exposed to the protease, the protease cleaves P in the activatable antibody.

In another preferred embodiment, R4 in the P is connected to a sulfhydryl group of the mutant CD47 antibody (Ab).

In the second aspect of the present invention, it provides an anti-CD47 antibody or an antigen-binding fragment thereof, wherein the anti-CD47 antibody comprises a heavy chain variable region and a light chain variable region,
(1) the heavy chain variable region comprises the following three heavy chain variable regions:
   HCDR1 SGYDMC (SEQ ID NO. 18),
   HCDR2 CIYTRSSGGTYYASWAKG (SEQ ID NO. 19),
   HCDR3 GRSSYRLNL (SEQ ID NO. 20), and
   the light chain variable region comprises the following three light chain variable regions:
   LCDR1 QASQSISNELS (SEQ ID NO. 21),
   LCDR2 RASTLES (SEQ ID NO. 22),
   LCDR3 QSTFYGDYGNA (SEQ ID NO. 23); or
(2) the heavy chain variable region comprises the following three heavy chain variable regions:
   HCDR1 SGYDMC (SEQ ID NO. 25),
   HCDR2 CIYTRSSGGTYYASWAKG (SEQ ID NO. 26),
   HCDR3 GRSSYRLNL (SEQ ID NO. 27), and
   the light chain variable region comprises the following three light chain variable regions:
   LCDR1 QASQSISNELS (SEQ ID NO. 28),
   LCDR2 RASTLES (SEQ ID NO. 29),
   LCDR3 QSTFYGDYGNA (SEQ ID NO. 30); or
(3) the heavy chain variable region comprises the following three heavy chain variable regions:
   HCDR1 SGYDMC (SEQ ID NO. 31),
   HCDR2 CIYTRSSGGTYYASWAKG (SEQ ID NO. 32),
   HCDR3 GRSSYRLNL (SEQ ID NO. 33), and
   the light chain variable region comprises the following three light chain variable regions:
   LCDR1 QASQSISNELS (SEQ ID NO. 34),
   LCDR2 RASTLES (SEQ ID NO. 35),
   LCDR3 QSTFYGDYGNA (SEQ ID NO. 36); or
(4) the heavy chain variable region comprises the following three heavy chain variable regions:
   HCDR1 SGYDMC (SEQ ID NO. 37),
   HCDR2 CIYTRSSGGTYYASWAKG (SEQ ID NO. 38),
   HCDR3 GRSSYRLNL (SEQ ID NO. 39), and
   the light chain variable region comprises the following three light chain variable regions:
   LCDR1 QASQSISNELS (SEQ ID NO. 40),
   LCDR2 RASTLES (SEQ ID NO. 41),
   LCDR3 QSTFYGDYGNA (SEQ ID NO. 42); or
(5) the heavy chain variable region comprises the following three heavy chain variable regions:
   HCDR1 SGYDMC (SEQ ID NO. 43),
   HCDR2 CIYTRSSGGTYYASWAKG (SEQ ID NO. 44),
   HCDR3 GRSSYRLNL (SEQ ID NO. 45), and

   the light chain variable region comprises the following three light chain variable regions:
      LCDR1 QASQSISNELS (SEQ ID NO. 46),
      LCDR2 RASTLES (SEQ ID NO. 47),
      LCDR3 QSTFYGDYGNA (SEQ ID NO. 48);
   wherein any of the above amino acid sequences further comprises a derivative sequence optionally subjected to addition, deletion, modification and/or substitution of at least one amino acid, and capable of retaining CD47 binding affinity.

In another preferred embodiment, the wild-type CD47 antibody comprises a heavy chain variable region as set forth in SEQ ID NO. 1, and a light chain variable region as set forth in SEQ ID NO. 2.

In another preferred embodiment, the wild-type CD47 antibody comprises a heavy chain variable region as set forth in SEQ ID NO. 3, and a light chain variable region as set forth in SEQ ID NO. 4.

In another preferred embodiment, the wild-type CD47 antibody comprises a heavy chain variable region as set forth in SEQ ID NO. 5, and a light chain variable region as set forth in SEQ ID NO. 6.

In another preferred embodiment, the wild-type CD47 antibody comprises a heavy chain variable region as set forth in SEQ ID NO. 7, and a light chain variable region as set forth in SEQ ID NO. 8.

In another preferred embodiment, the wild-type CD47 antibody comprises a heavy chain variable region as set forth in SEQ ID NO. 9, and a light chain variable region as set forth in SEQ ID NO. 10.

In another preferred embodiment, the wild-type CD47 antibody comprises a heavy chain variable region having an amino acid sequence with at least 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO. 1, 3, 5, 7, or 9.

In another preferred embodiment, the wild-type CD47 antibody comprises a light chain variable region having at least 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO. 2, 4, 6, 8, or 10.

In another preferred embodiment, the anti-CD47 antibody is a humanized antibody.

In another preferred embodiment, the heavy chain variable region of the humanized anti-CD47 antibody has an amino acid sequence selected from the group consisting of: SEQ ID NO. 11, 12, 13, and 14.

In another preferred embodiment, the light chain variable region of the humanized anti-CD47 antibody has an amino acid sequence selected from the group consisting of: SEQ ID NO. 15, 16, and 17.

In another preferred embodiment, the antibody comprises a heavy chain and a light chain.

In another preferred embodiment, the heavy chain further comprises a heavy chain constant region.

In another preferred embodiment, the heavy chain constant region is of human or murine origin.

In another preferred embodiment, the heavy chain constant region is a human antibody heavy chain IgG1 constant region.

In another preferred embodiment, the light chain further comprises a light chain constant region.

In another preferred embodiment, the light chain constant region is of human or murine origin.

In another preferred embodiment, the light chain constant region is a human antibody light chain kappa constant region.

In the third aspect of the present invention, it provides a recombinant protein, comprising:
(1) the mutant CD47 antibody of the first aspect of the present invention or the modified mutant CD47 antibody, or the anti-CD47 antibody of the second aspect of the present invention;
(2) an optional polypeptide molecule or fragment with therapeutic function; and/or
(3) an optional protein domain that assists in improving the physicochemical and pharmaceutical characteristics of a molecule.

In another preferred embodiment, the recombinant protein further comprises: (4) an optional tag sequence that assists in expression and/or purification.

In another preferred embodiment, the tag sequence is selected from the group consisting of: a 6His tag, a GGGS sequence, a FLAG tag.

In another preferred embodiment, the recombinant protein is a monomer, a dimer, or a multimer.

In another preferred embodiment, the polypeptide molecule or fragment with therapeutic function includes, but is not limited to: a polypeptide molecule or fragment targeting a target selected from the group consisting of: Her2, DLL3, CLDN6, EGFR, TGFβ, BCMA, B7H6, GUCY2C, CD478, CD123, CD19, CD20, CD22, B7-H3, GPC3, CD73, PMSA, CD28, 4-1BB, OX40, CD40, CD27, CTLA4, PD1, PDL1, BCMA, GLP-1, Trop2, TIGIT, LAG-3, FGL1, TLR7.

In another preferred embodiment, the recombinant protein (or polypeptide) comprises a fusion protein.

In another preferred embodiment, the fusion protein comprises a multispecific antibody, a chimeric antibody.

In another preferred embodiment, the functional domain that enhances the physicochemical properties or drugability of a protein comprises an Fc region, an anti-albumin nanobody (HLE), an albumin-binding domain (ABD).

In the fourth aspect of the present invention, it provides an isolated polynucleotide encoding the mutant CD47 antibody of the first aspect of the present invention or the anti-CD47 antibody of the second aspect of the present invention.

In the fifth aspect of the present invention, it provides a vector comprising the polynucleotide of the fourth aspect of the present invention.

In the sixth aspect of the present invention, it provides a genetically engineered host cell, wherein the host cell comprises the vector of the fifth aspect of the present invention or has the polynucleotide of the fourth aspect of the present invention integrated into its genome.

In the seventh aspect of the present invention, it provides a pharmaceutical composition, comprising:
(I) the mutant CD47 antibody of the first aspect of the present invention or the modified mutant CD47 antibody, or the anti-CD47 antibody of the second aspect of the present invention; and
(II) a pharmaceutically acceptable carrier.

In another preferred embodiment, the pharmaceutical composition further comprises:
(b) another biologically active drug, such as a drug for treating tumors.

In another preferred embodiment, the pharmaceutical composition comprises a single-agent drug, a combination drug, or a synergistic drug.

In another preferred embodiment, the mode of administration of the pharmaceutical composition is selected from the group consisting of: subcutaneous injection, intradermal injection, intramuscular injection, intravenous injection, intraperitoneal injection, microneedle injection, oral administration, oral/nasal spray and aerosol inhalation.

In another preferred embodiment, the mode of administration of the pharmaceutical composition comprises co-culturing the pharmaceutical composition with immune cells (e.g., dendritic cells, natural killer cells, lymphocytes, monocytes/macrophages, granulocytes, etc.), followed by isolation of the immune cells for *in vivo* reinfusion.

In another preferred embodiment, the dosage form of the pharmaceutical composition is selected from the group consisting of: liquid, solid, and gel state.

In another preferred embodiment, the pharmaceutical composition is used for anti-tumor therapy.

In the eighth aspect of the present invention, it provides an immunoconjugate, comprising:
(a) the mutant CD47 antibody of the first aspect of the present invention or the modified mutant CD47 antibody, or the anti-CD47 antibody of the second aspect of the present invention; and
(b) a coupling moiety selected from the group consisting of: a detectable label, a drug, a toxin, a cytokine, a radionuclide, an enzyme, and a combination thereof.

In another preferred embodiment, the components (a) and (b) are operably linked.

In another preferred embodiment, the coupling moiety is selected from: a fluorescent or luminescent label, a radioactive label, an MRI (magnetic resonance imaging) or CT (computed tomography) contrast agent, or an enzyme capable of producing a detectable product, a radionuclide, a biological toxin, a cytokine (e.g., IL-2, etc.), an antibody, an antibody Fc fragment, an antibody scFv fragment, a gold nanoparticle/nanorod, a viral particle, a liposome, a magnetic nanoparticle, a prodrug-activating enzyme (e.g., DT-diaphorase (DTD) or biphenyl hydrolase-like protein (BPHL)), a chemotherapeutic agent (e.g., cisplatin), or any form of nanoparticles, etc.

In the ninth aspect of the present invention, it provides a use of the mutant CD47 antibody of the first aspect of the present invention or the modified mutant CD47 antibody, the anti-CD47 antibody of the second aspect of the present invention, or the immunoconjugate of the eighth aspect of the present invention, for the preparation of (a) a detection reagent or kit; and/or (b) a pharmaceutical composition for preventing and/or treating cancer or a tumor or an autoimmune disease.

In the tenth aspect of the present invention, it provides a method for preventing or treating a tumor, comprising administering to a subject in need thereof the mutant CD47 antibody of the first aspect of the present invention or the modified mutant CD47 antibody, the pharmaceutical composition of the seventh aspect of the present invention, or the immunoconjugate of the eighth aspect of the present invention.

In another preferred embodiment, the tumor includes, but is not limited to: breast cancer, liver cancer, gastric cancer, large intestine cancer, leukemia, lung cancer, renal tumor, small intestine cancer, prostate cancer, colorectal cancer, prostate cancer, cervical cancer, lymphoma, bone cancer, adrenal tumor, or bladder tumor.

It should be understood that, within the scope of the present invention, the above-described technical features of the present invention and the technical features specifically described below (such as in the examples) can be combined with each other to form new or preferred technical solutions. Due to space limitations, they will not be described in detail here.

### DESCRIPTION OF THE DRAWINGS

Figure 1 shows the aligned sequences of the light chain and heavy chain variable regions of each anti-CD47 rabbit antibody.
Figure 2 shows the FACS binding assay of 5 strains of CD47 antibodies with CHOK1-CD47 cells.
Figure 3 shows the ELISA binding assay of CD47 antibodies with CD47 antigen.
Figure 4 shows the FACS binding assay of CD47 antibodies with CHOK1-CD47 cells.
Figure 5 shows the FACS binding assay of CD47 TMEAbody on Raji cells.
Figure 6 shows the assay of CD47 TMEAbody blocking the binding to SIRPα on Raji cells.
Figure 7 shows the blocking of CD47 antibody binding to red blood cells by CD47 TMEAbody.
Figure 8 shows the RBC agglutination assay of CD47 TMEAbody.
Figure 9 shows the ADCC activity of CD47 TMEAbody on Raji cells.
Figure 10 shows the FACS binding assay of different humanized CD47 antibodies with CHOK1-CD47 cells.
Figure 11 shows the *in vivo* pharmacodynamic assay of CD47 TMEAbody in a mouse tumor (Raji) model.
Figure 12 shows the *in vivo* pharmacodynamic assay of CD47 TMEAbody in a mouse tumor (SHP-77) model.
Figure 13 shows the *in vivo* pharmacodynamic assay of another CD47 TMEAbody in a mouse tumor (SHP-77) model.
Figure 14 shows the analysis result graph of the changes in the numbers of red blood cells (RBC) and platelets (PLT) in hSIRPA/hCD47 mice over days of antibody administration.

### DETAILED DESCRIPTION

Through extensive and in-depth research, the inventors unexpectedly discovered for the first time that a mutant CD47 antibody obtained by mutating specific amino acids in the CDRs of the variable region of a wild-type CD47 antibody to Cys, and a modified mutant CD47 antibody obtained by chemically conjugating a blocker thereto, can reduce the binding activity to CD47 and achieve specific activation in the tumor microenvironment; the modified mutant CD47 antibody can be activated in the tumor microenvironment and stimulate anti-tumor immune responses, while exhibiting good safety. The present invention has been accomplished on this basis.

### TERMS

Unless defined otherwise, all technical and scientific terms used herein have the same meaning as commonly understood by one of ordinary skill in the art to which this invention belongs.

The term "about" can refer to a value or composition that is within an acceptable error range for the particular value or composition as determined by one of ordinary skill in the art, which will depend in part on how the value or composition is measured or determined.

As used herein, the terms "comprising" or "including (containing)" may be open, semi-closed, or closed. In other words, the terms also include "substantially consisting of" or "consisting of".

### Antibody

Generally, "antibodies" are also called "immunoglobulins" and can be natural or conventional antibodies, in which two heavy chains are connected to each other by disulfide bonds and each heavy chain is connected to a light chain by a disulfide bond. There are two types of light chains, λ (l) and κ (k). There are five major heavy chain species (or isotypes), which determine the functional activity of the antibody molecule: IgM, IgD, IgG, IgA and IgE. Each chain contains different sequence domains. The light chain includes two domains or regions, the variable domain (VL) and the constant domain (CL). The heavy chain includes four domains, the heavy chain variable region (VH) and three constant regions (CH1, CH2 and CH3, collectively referred to as CH). The variable regions of both the light chain (VL) and the heavy chain (VH) determine the binding recognition and specificity for the antigen. The constant domain (CL) of the light chain and the constant region (CH) of the heavy chain confer important biological properties such as antibody chain binding, secretion, transplacental mobility, complement binding and binding to Fc receptors (FcR). The Fv fragment is the N-terminal portion of the immunoglobulin Fab fragment and is composed of the variable portion of one light chain and one heavy chain. The specificity of an antibody depends on the structural complementarity between the antibody binding site and the antigenic determinant region. The antibody binding site is composed primarily of residues from the hypervariable region or complementarity determining region (CDR). Occasionally, residues from the non-hypervariable or framework region (FR) influence the overall domain structure and, in turn, the binding site. The complementarity determining region or CDR refers to the amino acid sequence that collectively defines the binding affinity and specificity of the native Fv region of the natural immunoglobulin binding site. The light and heavy chains of immunoglobulins each have three CDRs, separately referred to as CDR1-L, CDR2-L, CDR3-L and CDR1-H, CDR2-H, CDR3-H. A conventional antibody antigen binding site therefore includes six CDRs, comprising a set of CDRs from the v region of each heavy and light chain.

As used herein, the terms "single-domain antibody" and "nanobody" have synonymous meanings and refer to the construction of a single-domain antibody consisting solely of a single heavy chain variable region by cloning the variable region of an antibody heavy chain. This is the smallest fully functional antigen-binding fragment. Typically, antibodies naturally lacking the light chain and heavy chain constant region 1 (CH1) are first obtained, and then the variable region of the antibody heavy chain is cloned to construct a single-domain antibody consisting solely of a single heavy chain variable region.

As used herein, the term "variable" refers to certain parts of the variable region in an antibody that are different in sequence, which form the binding and specificity of various specific antibodies to their specific antigens. However, variability is not evenly distributed throughout the variable region of an antibody. It is concentrated in three segments called complementary determining regions (CDRs) or hypervariable regions in the light and heavy chain variable regions. The more conserved parts of the variable region are called framework regions (FRs). The variable regions of natural heavy and light chains each contain four FR regions, which are generally in a β-sheet configuration and are connected by three CDRs that form a connecting loop, and in some cases can form a partial β-sheet structure. The CDRs in each chain are closely together through the FR regions and form the antigen-binding site of the antibody together with the CDRs of the other chain (see Kabat et al., NIH Publ. No. 91-3242, Volume 1, pages 647-669 (1991)). The constant regions do not directly participate in the binding of the antibody to the antigen, but they exhibit different effector functions, such as participating in the antibody's antibody-dependent cytotoxicity.

As used herein, the term "framework region (FR)" refers to the amino acid sequence inserted between the CDRs, that is, it refers to those portions of the light and heavy chain variable regions of immunoglobulins that are relatively conserved between different immunoglobulins in a single species. The light and heavy chains of immunoglobulins each have four FRs, referred to as FR1-L, FR2-L, FR3-L, FR4-L and FR1-H, FR2-H, FR3-H, FR4-H, respectively. Accordingly, the light chain variable domain can be referred to as (FR1-L)-(CDR1-L)-(FR2-L)-(CDR2-L)-(FR3-L)-(CDR3-L)-(FR4-L), and the heavy chain variable domain can be represented as (FR1-H)-(CDR1-H)-(FR2-H)-(CDR2-H)-(FR3-H)-(CDR3-H)-(FR4-H). Preferably, the FR of the present invention is a human antibody FR or a derivative thereof, wherein the derivative of the human antibody FR is substantially identical to the naturally occurring human antibody FR, i.e., the sequence identity reaches 85%, 90%, 95%, 96%, 97%, 98% or 99%.

"Percent (%) amino acid sequence identity" and "homology" with respect to peptide, polypeptide or antibody sequences are defined as the percentage of amino acid residues in the candidate sequence that are identical to the amino acid residues in the specific peptide or polypeptide sequence, after aligning the candidate sequence with the specific peptide or polypeptide sequence (and introducing gaps, if necessary) to achieve the maximum percent sequence identity, and not considering any conservative substitutions as part of the sequence identity. Sequence alignment for determination of percent amino acid sequence identity can be performed using various methods in the art, for example, using publicly available computer software such as BLAST, BLAST-2, ALIGN or MEGALIGN^{™} (DNASTAR) software.

Knowing the amino acid sequence of the CDRs, one skilled in the art can easily determine the framework regions FR1-L, FR2-L, FR3-L, FR4-L and/or FR1-H, FR2-H, FR3-H, FR4-H.

As used herein, the term "human framework region" is a framework region that is substantially identical (about 85% or more, specifically 90%, 95%, 97%, 99% or 100%) to the framework region of a naturally occurring human antibody.

As used herein, the term "monoclonal antibody" or "mAb" refers to an antibody molecule with a single amino acid composition directed against a specific antigen, and should not be construed as requiring the production of the antibody by any particular method. Monoclonal antibodies can be produced by a single clone of a B cell or hybridoma, but can also be recombinant, i.e., produced by protein engineering.

As used herein, the term "antigen" or "target antigen" refers to a molecule or portion of a molecule that can be bound by an antibody or antibody-like binding protein. The term further refers to a molecule or portion of a molecule that can be used in an animal to produce an antibody that can bind to an epitope of the antigen. A target antigen may have one or more epitopes. For each target antigen recognized by an antibody or by an antibody-like binding protein, the antibody-like binding protein can compete with an intact antibody that recognizes the target antigen.

As used herein, the term "affinity" is theoretically defined by the equilibrium association between an intact antibody and an antigen. The affinity of the bispecific antibodies of the present invention can be assessed or determined by KD values (dissociation constants) (or other assays), such as bio-layer interferometry (BLI), using a FortebioRed 96 instrument.

As used herein, the term "EC₅₀ ", also known as half-maximal effect concentration, refers to the antibody concentration that causes 50% of the maximum effect.

As used herein, the term "knobs-into-holes (KIH)" refers to the currently mature Fc segment heterodimerization technology. For example, the small threonine (T) at position 366 of the CH3 region of an antibody heavy chain is replaced with a larger tryptophan (W) to form a knob structure (T366W), and the larger tyrosine (Y) at position 407 of the CH3 region of another antibody heavy chain is mutated to a smaller threonine (T) to form a hole structure (Y407T). Relying on the reduced steric hindrance effect after the mutation and the covalent disulfide bond formed in the hinge region, the heterologous heavy chain dimerization is promoted, which is the KIH structure. Currently, the more commonly used knob-into-hole structures in the Fc segment include improving the Y407T single point mutation of the heavy chain hole to a three-point mutation (T366S, L368A and Y407V) to obtain a stable "1+3" knob-into-hole structure, or further adding an S354C site mutation to the heavy chain knob and an S349C site mutation to the heavy chain hole.

### Tumor microenvironment-activating antibody

As used herein, the terms "masked antibody of the present invention" "tumor microenvironment activated antibody of the present invention" and "TMEAbody (Tumor microenvironment activated antibody) of the present invention" can be used interchangeably and refer to derivative antibodies comprising the modified mutant CD47 antibody or mutant CD47 antibody (CD47 antibody mutant) of the first aspect of the present invention, wherein the derivative antibody is a modified biomolecule (including a blocker and a linker peptide cleavable under pathological conditions) that is activated only in a pathological microenvironment, such as a tumor microenvironment or inflammatory site. It can improve the targeting efficacy of biomolecules by releasing them at the pathological site, overcome drug resistance, and reduce toxicity (e.g., the toxicity of the CD47 antibody).

It should be understood that the masked antibodies of the present invention include: masked antibodies that have not been activated in a pathological microenvironment and masked antibodies that have been activated in a pathological microenvironment (for example, the cleavable linker peptide is cut by a proteolytic enzyme in a tumor microenvironment to release the blocker and obtain an activated biological molecule).

Preferably, the modified mutant CD47 antibody of the present invention has reduced binding affinity to CD47 in the inactivated state, but *in vitro* experiments show that it still maintains a certain CD47 binding ability; and, in the activated state, its CD47 binding ability is restored to a level equivalent to or better than the wild-type CD47 antibody.

In tumor patients, tumor cells or antigen-presenting cells (APCs) carrying tumor antigens partially or completely inhibit the host's immune killing of tumors by binding to T cells. However, the tumor microenvironment-activated antibodies of the present invention are activated and released in a pathological microenvironment by proteolytic enzymes, particularly asparagine endopeptidases or granzymes, or under acidic conditions.

The antibody used in the present invention can be an antibody known in the art or a functional fragment thereof. For example, the antibody or its functional fragment can be selected from: anti-Her2 antibody, anti-EGFR antibody, anti-VEGFR antibody, anti-CD20 antibody, anti-CD473 antibody, anti-PD-L1 antibody, anti-PD-1 antibody, anti-CTLA-4 antibody, anti-TNFα antibody, anti-CD28 antibody, anti-4-1BB antibody, anti-OX40 antibody, anti-GITR antibody, anti-CD27 antibody, anti-b-CD40 antibody, or anti-ICOS antibody, anti-CD25 antibody, anti-CD470 antibody, anti-CD47 antibody, anti-CD22 antibody, anti-CCR4 antibody, anti-CD478 antibody, anti-CD52 antibody. body, anti-complement C5 antibody, anti-RSV F protein, anti-GD2 antibody, anti-GITR antibody, anti-glycoprotein receptor lib/Illa antibody, anti-ICOS antibody, anti-IL2R antibody, anti-LAG3 antibody, anti-Integrin α4 antibody, anti-IgE antibody, anti-PDGFRa antibody, anti-RANKL antibody, anti-SLAMF7 antibody, anti-LTIGIT antibody, anti-TIM-3 antibody, anti-VEGFR2 antibody, anti-VISTA antibody.

Within the scope of this disclosure, the meaning and structure of cytokines are as generally defined in the art. Cytokines generally refer to a class of small proteins with a wide range of biological activities, whose synthesis and secretion are achieved by stimulating immune cells, such as monocytes, macrophages, T cells, B cells, NK cells, etc., or non-immune cells, such as endothelial cells, epidermal cells, fibroblasts, etc. Cytokines can regulate cell proliferation, differentiation, function, and immune response by binding to corresponding receptors. Suitable cytokines include interleukins, interferons, tumor necrosis factors, colony-stimulating factors, chemokines, and growth factors.

Exemplary cytokines include, but are not limited to, IL-2, IL-7, IL-10, IL-12, IL-15, IL-21, IFN-α, IFN-β, IFN-γ, G-CSF, GM-CSF, TNF-α, TRAP, and TRAIL.

In a specific embodiment, it provides a CD47 TMEAbody tumor microenvironment activated antibody.

Furthermore, the modified mutant CD47 antibody has the structure shown below:

Ab-(P-R1)n

wherein,
Ab is a CD47 antibody mutant;
R1 is each independently a blocker;
P is a cleavable linker peptide;
n is a positive integer of 1 to 5;
"-" represents a chemical bond, a linker, or a connector.

Preferably, the blocker R4 is site-specifically linked to an amino acid site selected from the following group of sites in the CD47 antibody mutant via a cleavable linker peptide P.

Preferred mutation sites include Y61C in VH and Y49C in VL.

Within the scope of the present disclosure, one or more (e.g., up to five, or up to three) amino acid residues in the amino acid sequence of the D1 biomolecule are mutated to cysteine residues, and covalently linked to the blocker (P-R1) via a thiol bond of the cysteine residues. For example, one or two amino acid residues of interest in the biomolecule of interest are mutated to cysteine residues to link to the functional group. For an antibody, the mutation site can be in the complementarity determining region or the non-complementarity determining region of the variable region.

Preferably, the mutation is a substitution mutation. More preferably, the mutation occurs in the variable region of the antibody light chain. Generally, a mutant can be prepared first and then tested for its binding activity to the corresponding antigen. If the mutant retains 70% or more, preferably 80% or more, more preferably 90% or more binding activity compared to the wild-type antibody, it is considered that the amino acid residue at the mutation position can be mutated to cysteine to be covalently linked to the functional group. Alternatively, in certain embodiments, if the conjugate produced by connecting the mutant to R4 retains 80% or more, preferably 90% or more, more preferably 95% or more binding activity, it is considered that the amino acid residue at the mutation position can be mutated to cysteine to be covalently linked to the functional group.

As used herein, the term "linker peptide" refers to one or more amino acid residues inserted into an immunoglobulin domain to provide sufficient mobility for the light and heavy chain domains to fold into an exchange dual variable region immunoglobulin.

Suitable linker examples include a single glycine (Gly) residue or a single serine (Ser) residue, and the identity and sequence of amino acid residues in the linker can vary with the type of secondary structural elements desired to be achieved in the linker. A preferred linker is as follows: GS, (G4S)n, where n is an integer of 1 to 4.

The antibodies of the present invention include not only intact antibodies, but also immunologically active antibody fragments or fusion proteins formed by antibodies with other sequences. Therefore, the present invention also includes fragments, derivatives, and analogs of the antibodies. As used herein, the terms "fragment", "derivative", and "analog" refer to polypeptides that substantially retain the same biological function or activity as the antibodies of the present invention. The polypeptide fragments, derivatives, or analogs of the present invention can be (i) polypeptides having one or more conservative or non-conservative amino acid residues (preferably conservative amino acid residues) substituted, where such substituted amino acid residues may or may not be encoded by the genetic codes, or (ii) polypeptides having a substituent group in one or more amino acid residues, or (iii) polypeptides formed by fusion of a mature polypeptide with another compound (such as a compound that extends the half-life of the polypeptide, such as polyethylene glycol), or (iv) polypeptides formed by fusion of additional amino acid sequences to the polypeptide sequence (such as a leader sequence or secretory sequence, a sequence used to purify the polypeptide, a proprotein sequence, or a fusion protein formed with a 6His tag). Based on the teachings herein, these fragments, derivatives, and analogs are well known to those skilled in the art.

The antibodies of the present invention refer to antibodies with anti-CD47 properties. The term also includes variant forms comprising two antibodies of the above structures that have the same functions as the antibodies of the present invention. These variant forms include (but are not limited to): deletion, insertion and/or substitution of one or more (usually 1-50, preferably 1-30, more preferably 1-20, and most preferably 1-10) amino acids, and addition of one or more (usually within 20, preferably within 10, and more preferably within 5) amino acids to the C-terminus and/or N-terminus. For example, in the art, substitution with amino acids having similar properties generally does not change the function of the protein. For another example, addition of one or more amino acids to the C-terminus and/or N-terminus generally does not change the function of the protein. The term also includes active fragments and active derivatives of the antibodies of the present invention.

Variant forms of the antibody include: homologous sequences, conservative variants, allelic variants, natural mutants, induced mutants, proteins encoded by DNA that can hybridize with the encoding DNA of the antibody of the present invention under high or low stringency conditions, and polypeptides or proteins obtained using antiserum against the antibody of the present invention.

In the present invention, "conservative variants of the antibodies of the present invention" refer to polypeptides in which no more than 10, preferably no more than 8, more preferably no more than 5, and most preferably no more than 3 amino acids are substituted with amino acids having similar properties, compared to the amino acid sequence of the antibodies of the present invention. These conservative variant polypeptides are preferably generated by making amino acid substitutions according to Table A.

**Table A**

| Original Residue | Representative Substitution | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Encoding nucleic acid and expression vector

The present invention also provides polynucleotide molecules encoding the above-mentioned antibodies or fragments thereof or fusion proteins thereof. The polynucleotides of the present invention may be in the form of DNA or RNA. DNA forms include cDNA, genomic DNA, or artificially synthesized DNA. DNA may be single-stranded or double-stranded. DNA may be a coding strand or a non-coding strand. Polynucleotides encoding the mature polypeptides of the present invention include: a coding sequence that encodes only the mature polypeptide; a coding sequence for the mature polypeptide and various additional coding sequences; a coding sequence for the mature polypeptide (and optional additional coding sequences) and non-coding sequences.

The term "polynucleotide encoding a polypeptide" may include a polynucleotide encoding the polypeptide, or may also include additional coding and/or non-coding sequences.

The nucleic acids (and nucleic acid combinations) of the present invention can be used to produce the recombinant antibodies of the present invention in a suitable expression system.

The present invention also relates to polynucleotides that hybridize to the above-mentioned sequences and there are at least 50%, preferably at least 70%, and more preferably at least 80% identity between the two sequences. The present invention particularly relates to polynucleotides that hybridize to the polynucleotides of the present invention under stringent conditions. In the present invention, "stringent conditions" refer to: (1) hybridization and elution at relatively low ionic strength and relatively high temperature, such as 0.2×SSC, 0.1% SDS, 60°C; or (2) the addition of a denaturing agent during hybridization, such as 50% (v/v) formamide, 0.1% calf serum/0.1% Ficoll, 42 °C; or (3) hybridization occurs only when the identity between the two sequences is at least 90%, more preferably at least 95%. Furthermore, the polypeptide encoded by the hybridizable polynucleotide has the same biological function and activity as the mature polypeptide.

The full-length nucleotide sequence of the antibody of the present invention or its fragments can generally be obtained by PCR amplification, recombinant methods, or artificial synthesis methods. One feasible method is to synthesize the relevant sequence by artificial synthesis, especially when the fragment length is relatively short. Generally, by first synthesizing multiple small fragments and then ligating them, a very long fragment of sequence can be obtained. In addition, the coding sequence of the heavy chain can be fused with an expression tag (such as 6His) to form a fusion protein.

Once the relevant sequence is obtained, recombinant methods can be used to obtain it in large quantities. This is typically accomplished by cloning it into a vector, transferring it into cells, and then isolating the relevant sequence from the proliferated host cells using conventional methods. The biomolecules (nucleic acids, proteins, etc.) referred to in the present invention include biomolecules in isolated form.

Currently, DNA sequences encoding proteins of the present invention (or fragments thereof, or derivatives thereof) can be obtained entirely by chemical synthesis. This DNA sequence can then be introduced into various existing DNA molecules (or vectors) and cells known in the art. In addition, mutations can also be introduced into protein sequences of the present invention by chemical synthesis.

The present invention also relates to vectors comprising the above-mentioned appropriate DNA sequence and appropriate promoter or control sequence. These vectors can be used to transform appropriate host cells to enable them to express proteins.

Host cells can be prokaryotic cells, such as bacterial cells; lower eukaryotic cells, such as yeast cells; or higher eukaryotic cells, such as mammalian cells. Representative examples include: *Escherichia coli*, *Streptomyces*; bacterial cells of *Salmonella typhimurium*; fungal cells, such as yeast; insect cells such as Drosophila S2 or Sf9; and animal cells such as CHO, COS7, and 293 cells etc.

Transformation of host cells with recombinant DNA can be performed using conventional techniques well known to those skilled in the art. When the host is a prokaryotic organism such as *Escherichia coli*, competent cells capable of absorbing DNA can be harvested after the exponential growth phase and treated using the CaCl₂ method, using procedures well known in the art. Another method is to use MgCl₂. If desired, transformation can also be performed using electroporation. When the host is a eukaryotic organism, the following DNA transfection methods can be used: calcium phosphate coprecipitation, conventional mechanical methods such as microinjection, electroporation, liposome packaging, etc.

The obtained transformants can be cultured using conventional methods to express the polypeptide encoded by the gene of the present invention. Depending on the host cell used, the culture medium used can be selected from various conventional culture media. Culture is carried out under conditions suitable for the growth of the host cells. After the host cells grow to an appropriate cell density, the selected promoter is induced using a suitable method (such as temperature conversion or chemical induction), and the cells are cultured for a period of time.

Under early culture conditions, the expression level of bispecific antibodies can reach 3.9 g/L, the purity is above 97%, and lactic acid can be metabolized well during the culture process.

The recombinant polypeptide in the above method can be expressed intracellularly, on the cell membrane, or secreted extracellularly. If necessary, the recombinant protein can be isolated and purified by various separation methods utilizing its physical, chemical, and other properties. These methods are well known to those skilled in the art. Examples of these methods include, but are not limited to, conventional renaturation treatment, treatment with a protein precipitant (salting out method), centrifugation, osmotic sterilization, ultrafiltration, ultracentrifugation, molecular sieve chromatography (gel filtration), adsorption chromatography, ion exchange chromatography, high performance liquid chromatography (HPLC), and various other liquid chromatography techniques and combinations of these methods.

The bispecific antibodies of the present invention can be used alone or in combination with a detectable marker (for diagnostic purposes), a therapeutic agent, or any combination thereof.

Detectable labels for diagnostic purposes include, but are not limited to, fluorescent or luminescent labels, radioactive labels, MRI (magnetic resonance imaging) or CT (computed tomography) contrast agents, or enzymes capable of producing a detectable product.

Therapeutic agents that can be combined or coupled with the antibodies of the present invention include but are not limited to: 1. radionuclides; 2. biological toxins; 3. cytokines such as IL-2; 4. gold nanoparticles/nanorods; 5. viral particles; 6. liposomes; 7. nanomagnetic particles; 8. tumor therapeutic agents (e.g., cisplatin) or any form of anti-tumor drugs, etc.

### Pharmaceutical composition

The present invention also provides a composition. Preferably, the composition is a pharmaceutical composition, which contains the bispecific antibody or its active fragment or its fusion protein described above, and a pharmaceutically acceptable carrier. Generally, these substances can be formulated in a non-toxic, inert and pharmaceutically acceptable aqueous carrier medium, wherein the pH is generally about 5-8, preferably about 6-8, although the pH value may vary depending on the properties of the formulated substance and the condition to be treated. The formulated pharmaceutical composition can be administered by conventional routes, including (but not limited to): intravenous injection, intravenous drip, subcutaneous injection, local injection, intramuscular injection, intratumoral injection, intraperitoneal injection (such as intraperitoneal administration), intracranial injection, or intracavitary injection.

The pharmaceutical composition of the present invention contains a safe and effective amount (such as 0.001-99 wt%, preferably 0.01-90 wt%, more preferably 0.1-80 wt%) of the above-mentioned nanoantibody (or its conjugate) of the present invention and a pharmaceutically acceptable carrier or excipient. Such carriers include (but are not limited to): saline, buffer, glucose, water, glycerol, ethanol, and combinations thereof. The pharmaceutical preparation should match the mode of administration. The pharmaceutical composition of the present invention can be prepared in the form of an injection, for example, by conventional methods using physiological saline or an aqueous solution containing glucose and other adjuvants. Pharmaceutical compositions such as injections and solutions are preferably manufactured under sterile conditions. The dosage of the active ingredient is a therapeutically effective amount, for example, about 10 micrograms/kg body weight to about 50 mg/kg body weight per day. In addition, the polypeptide of the present invention can also be used with other therapeutic agents.

In the present invention, the bispecific antibody can be used alone, and the dosage regimen can be adjusted to achieve the optimal desired response, for example, a single administration, or multiple administrations over a period of time, or the dosage can be proportionally reduced or increased depending on the urgency of the therapeutic situation.

When using a pharmaceutical composition, a safe and effective amount of the immunoconjugate is administered to a mammal, wherein the safe and effective amount is generally at least about 10 µg/kg body weight, and in most cases does not exceed about 50 mg/kg body weight. Preferably, the dose is about 10 µg/kg body weight to about 10 mg/kg body weight. Of course, the specific dose should also take into account factors such as the route of administration and the patient's health status, which are all within the skill of a skilled physician.

**The main advantages of the present invention include:**
(1) The modified mutant CD47 antibody in the present invention exhibits excellent safety, with no impact on red blood cell aggregation, effectively reducing the toxic side effects of the antibody.
(2) The CD47 TMEAbody in its blocked state largely prevents the binding of the CD47 antibody to CD47 or its ADCC activity, whereas the activated CD47 TMEAbody can restore its binding affinity.

The present invention will be further described below in conjunction with specific examples. It should be understood that these examples are intended to illustrate the present invention only and are not intended to limit the scope of the present invention. The experimental methods in the following examples, for which specific conditions are not specified, are generally performed under conventional conditions, such as those described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

### Example 1: Preparation of rabbit anti-CD47 monoclonal antibody

New Zealand white rabbits were immunized with human CD47. Individual B cells were isolated using an optimized protocol from published literature (Offner et al. PLoS ONE 9(2), 2014). Human CD47+ B cells were sorted into single wells by IgG+ direct flow cytometry. B cell culture supernatants were screened for binding to human CD47 by enzyme-linked immunosorbent assay (ELISA), and the B cells were lysed and stored at -80°C.

The light and heavy chain variable regions of the CD47-specific B cells were amplified by PCR and cloned into expression vectors as described in the published literature (Offner et al. PLoS ONE 9(2), 2014). Each recombinant rabbit monoclonal antibody was expressed in Expi293 cells and purified with Protein A. The purified anti-CD47 antibodies were then subjected to functional identification.

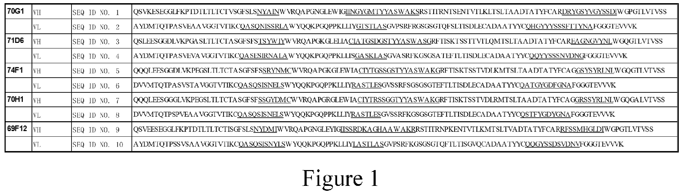

The aligned sequences of the light and heavy chain variable regions for each anti-CD47 rabbit antibody are shown in Figure 1.

The amino acid sequence of the heavy chain variable region (VH) of 70G1 as set forth in SEQ ID NO. 1

QSVKESEGGLFKPTDTLTLTCTVSGFSLSNYAINWVRQAPGNGLEWIGIINGYGMTY
YASWAKSRSTITRNTSENTVTLKLTSLTAADTATYFCARDRYGSYVGYSSDIWGPGTLVT
VSS

The amino acid sequence of the light chain variable region (VL) of 70G1 as set forth in SEQ ID NO. 2

AYDMTQTPASVEAAVGGTVTIKCQASQNISSRLAWYQQKPGQPPKLLIYGTSTLASG
VPSRFRGSGSGTQFSLTISDLECADAATYYCQHGYYYSSSFTTYNAFGGGTEVVVK

The amino acid sequence of the heavy chain variable region (VH) of 71D6 as set forth in SEQ ID NO. 3

QSLEESGGDLVKPGASLTLTCTASGFSFSTSYWIYWVRQAPGKGLELIACIATGSDGS
TYYASWASGRFTISKTSSTTVTLQMTSLTAADTATYFCAREAGNGVYNLWGQGTLVTVS
S

The amino acid sequence of the light chain variable region (VL) of 71D6 as set forth in SEQ ID NO. 4

AYDMTQTPASVEVAVGGTVTIKCQASESIRNALAWYQQKPGQPPKLLISGASKLASG
VASRFKGSGSATEFTLTISDLECADAATYYCQQYYSSSNVDNGFGGGTEVVVK

The amino acid sequence of the heavy chain variable region (VH) of 74F1 as set forth in SEQ ID NO. 5

QQQLEESGGDLVKPEGSLTLTCTASGFSFSSRYNMCWVRQAPGKGLEWIACIYTGSS
GSTYYASWAKGRFTISKTSSTVDLKMTSLTAADTATYFCAGGSYSYRLNLWGQGTLVTV
SS

The amino acid sequence of the light chain variable region (VL) of 74F1 as set forth in SEQ ID NO. 6

DVVMTQTPASVSTAVGGTVTIKCQASQSISNELSWYQQKPGQPPKLLIYRASTLESG
VSSRFSGSGSGTEFTLTISDLECADAATYYCQATGYGDFGNAFGGGTEVVVK

The amino acid sequence of the heavy chain variable region (VH) of 70H1 as set forth in SEQ ID NO. 7

QQQLEESGGGLVKPEGSLTLTCTASGFSFSSGYDMCWVRQAPGRGLEWIACIYTRSS
GGTYYASWAKGRFTISKTSSTVDLRMTSLTAADTATYFCAGGRSSYRLNLWGQGALVTV
SS

The amino acid sequence of the light chain variable region (VL) of 70H1 as set forth in SEQ ID NO. 8

DVVMTQTPSPVEAAVGGTVTIKCQASQSISNELSWYQQKPGQPPKLLIYRASTLESG
VSSRFKGSGSGTEFTLTISDLECADAATYYCQSTFYGDYGNAFGGGTEVVVK

The amino acid sequence of the heavy chain variable region (VH) of 69F12 as set forth in SEQ ID NO. 9

QSVEESEGGLFKPTDTLTLTCTISGFSLSNYDMIWVRQAPGNGLEYIGIISSRDKAGH
AAWAKRRSTITRNPKENTVTLKMTSLTVADTATYFCARRFSSMHGLDIWGPGTLVTVSS

The amino acid sequence of the light chain variable region (VL) of 69F12 as set forth in SEQ ID NO. 10

AYDMTQTPSSVSAAVGGTVTIKCQASQSISNYLSWYQQKPGQPPKLLIYLASTLASG
VPSRFKGSGSGTQFTLTISGVQCADAATYYCQQGYSSDSVDNVFGGGTEVVVK

### Example 2: FACS binding of CD47 antibodies to CHOK1-CD47 cells

The desired overexpressing cell line, CHOK1-CD47 (cultured in RPMI-1640 (ATCC Modification, Gibco, Cat# A10491-01) + 10% FBS (BI, Cat# 04-001-1A) + 1× P/S + 6 µg/ml Puromycin (Gibco, Cat# a1113803, 10 × 1 ml (10 mg/ml))), was expanded to the logarithmic phase. Cells were digested, collected, and pipetted to create a single-cell suspension, followed by centrifugation and removal of the supernatant. The cells were resuspended in FACS buffer (PBS + 2% FBS) and counted, and the cell density was adjusted to 2×10⁶/ml. 100 µl/well of this suspension was added to a 96-well plate (Corning, Cat# 3799). After centrifugation and removal of the supernatant, 100 µl/well of the antibody sample to be tested (initial concentration of 100 nM, with 5-fold serial dilutions) was added, mixed with the cells, and incubated at 4 °C for 1 hour. Cells were washed three times by centrifugation with FACS buffer. Then, 100 µl/well of Goat anti-Human IgG Fc Secondary Antibody, PE (eBioscience^{™}, Invitrogen, Cat# 12-4998-82, 1:200 dilution) was added and incubated at 4°C for 1 hour. After three washes with FACS buffer by centrifugation, the cells were resuspended in 200 µl/well of FACS buffer and analyzed using FACS (BECKMAN COULTER, CytoFLEX). Data were fitted and the results were analyzed using software (GraphPad Prism 8).

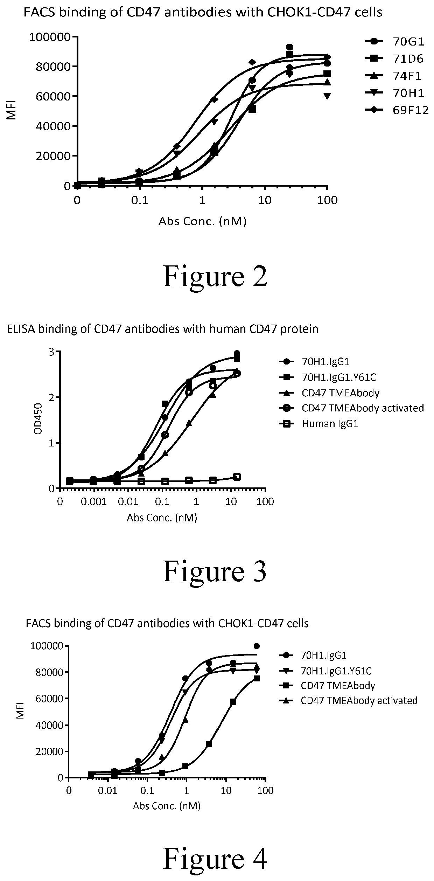

As shown in Figure 2, all five antibodies bound well to CHOK1-CD47 cells. The calculated EC50 (nM) values for 70G1, 71D6, 74F1, 70H1, and 69F12 were 2.88, 3.80, 2.82, 0.91, and 0.80, respectively.

### Example 3: Screening of 70H1 TMEAbody sites

In this example, 70H1 was selected as the parent antibody for TMEAbody site screening. Corresponding DNA coding sequences (codon-optimized) were synthesized to facilitate expression in a host.

The synthesized DNA was digested and ligated into a modified pTT5 vector (Biovector) to generate pTT5-anti-CD47. Using pTT5-anti-CD47 as a template, cysteine mutations were introduced. Primers were designed to replace the codon at the mutation position with a cysteine (Cys) codon. PCR was performed, and the mutated fragment was digested and constructed into the modified pTT5 vector.

HEK293T cells (Life Technologies) were used as the expression host. Before transfection, HEK293T cells were cultured in complete medium containing 10% FBS (Gibco) at 37 °C in a 5% CO₂ atmosphere. One day before transfection, cells were inoculated into 15 cm culture dishes at an appropriate density, and the medium was replaced with medium containing low-IgG FBS (Gibco). Six hours or one day post-transfection, the medium was replaced with Freestyle 293 medium (Gibco).

On the day of transfection, when the cells reached a certain confluency, plasmids expressing the target proteins were co-transfected into 293T cells using Lipofectamine 2000 (Life Technologies) and PEI (Sigma). The antibody expression plasmid includes the heavy and light chains of the antibody. Culture supernatants were harvested on day 4 and day 6 post-transfection. The expression and activity of the protein or antibody were detected, and the protein or antibody were purified.

The hypervariable regions within the variable domains of the antibody heavy and light chains constitute the antigen-binding site. Due to their structural complementarity with the antigenic epitope, these hypervariable regions are also referred to as complementarity-determining regions (CDRs).

In the present invention, the activity of each mutant with point mutation was compared to its parent antibody by ELISA. Specifically, 96-well plates were coated with the antibody antigen overnight. The plates were then blocked with 1% BSA blocker (Thermo Fisher) at 37 °C for 2 hours and washed with PBST for three times. Corresponding antibodies or mutants were added and allowed to bind at 37 °C for 1 hour, then the plates were washed with PBST for three times. HRP-conjugated anti-human IgG was added, incubated at 37 °C for 1 hour, and then the plates were washed with PBST for three times. TMB substrate (Solarbio., Inc.) was used to measure the absorbance at 450 nm.

The effect on the binding strength of the mutants was calculated as EC50 (post-mutation) / EC50 (wild-type).

Based on the method described above, the binding activities of anti-CD47 antibody 70H1 mutants with mutations at different positions, as shown in Table 1, were evaluated.

The amino acid sequence of the heavy chain variable region (VH) of 70H1: SEQ ID NO. 7

QQQLEESGGGLVKPEGSLTLTCTASGFSFSSGYDMCWVRQAPGRGLEWIACIYTSSG
GTYYASWAKGRFTISKTSSTVDLRMTSLTAADTATYFCAGGRSSYRLNLWGQGALVTVS
S

The amino acid sequence of the light chain variable region (VL) of 70H1: SEQ ID NO. 8

DVVMTQTPSPVEAAVGGTVTIKCQASQSISNELSWYQQKPGQPPKLLIYRASTLESG
VSSRFKGSGSGTEFTLTISDLECADAATYYCQSTFYGDYGNAFGGGTEVVVK

**Table 1: Effect of mutation positions in the antibody heavy chain variable region/light chain variable region on binding activity**

| CDR | Position | Original Amino Acid of Anti-CD47 Antibody | Mutated Amino Acid of Anti-CD47 Antibody | Mutant Binding Strength (EC50 post-mutation / EC50 wild-type) |
|---|---|---|---|---|
| HCDR1 | VH-33 | Y | C | 1 |
| HCDR2 | VH-55 | R | C | 1 |
| HCDR2 | VH-61 | Y | C | 1 |
| HCDR2 | VH-65 | W | C | 0.8 |
| HCDR3 | VH-105 | Y | C | 1 |
| HCDR3 | VH-107 | L | C | 1.2 |
| LCDR1 | VL-27 | Q | C | 1 |
| Light chain | VL-49 | Y | C | 0.9 |
| LCDR2 | VL-53 | T | C | 1 |
| LCDR3 | VL-92 | F | C | 1.3 |
| LCDR3 | VL-93 | Y | C | 1 |
| LCDR3 | VL-96 | Y | C | 1 |
| LCDR3 | VL-98 | N | C | 1 |

| | | | | |
|---|---|---|---|---|
| Among them, VL-49 is located in the FR region. | | | | |

### Example 4. The binding affinity of CD47 TMEAbody to CD47 is reduced and can be restored

Based on the structural studies and screening conducted by the present inventors, it was indicated that the VH-61 (Y-C) site exhibited an excellent blocking effect (this site corresponds to position 61 in VH3, which can be referred to the Y residue at position 61 underlined in the VH3 sequence shown in Table 6). In this example, the coding sequence for the corresponding CD47 antibody (70H1.IgG1) containing the VH-61 (Y61C mutation) was synthesized and expressed to obtain the recombinant antibody (70H1.IgG1.Y61C). Furthermore, the obtained CD47 antibody was conjugated with PEG (with a molecular weight approximately 20K or other molecular weights of PEG), thereby generating the CD47 TMEAbody.

### 4.1 ELISA detection of CD47 antibody

Recombinant human CD47 protein (DIMA BIOTECH, Cat# PME-100008) was diluted with PBS to a concentration of 1 µg/ml, coated onto ELISA plates with 100 µl/well, and incubated overnight at 4 °C. On the following day, the plates were washed with 300 µl/well of PBST for three times. 300 µl/well of blocking solution (PBST containing 1% BSA (W/W)) was added, and the plates were blocked at 37 °C for 2 hours. After the blocking solution was discarded, the plates were washed with PBST for three times. 100 µl/well of antibody (5-fold gradient dilution, 8 dilution points) was added and incubated at 37 °C for 1 hour. The antibody solution was then discarded, and the plates were washed with PBST for three times. Goat anti-Human IgG Fc Highly Cross-Adsorbed Secondary Antibody, HRP (Invitrogen, Cat# A18829, 1:5000 dilution, 100 µl/well) was added and incubated at 37 °C for 0.5 hours, followed by three washes with PBST. 100 µl/well of TMB chromogenic substrate (solarbio, Cat# PR1200) was added and incubated at 37 °C for 5 minutes, after which 50 µl/well of ELISA stop solution (solarbio, Cat# C1058) was added. The OD450 was read using a microplate reader (ELx800 Absorbance Microplate Reader, BioTek). Data analysis and curve fitting were performed using software (GraphPad Prism 8).

As shown in Figure 3 and Table 2.1, the binding affinity of the CD47 TMEAbody (70H1. IgG1 + 20K PEG) to the human CD47 protein was approximately 7-fold weaker compared to 70H1. This binding affinity was shown to be recoverable by legumain (also known as asparaginyl endopeptidase, AEP).

**Table 2.1**

| Antibody name | EC50 (nM) |
|---|---|
| 70H1.IgG1 | 0.12 |
| 70H1.IgG1.Y61C | 0.07 |
| CD47 TMEAbody | 0.72 |
| CD47 TMEAbody activated | 0.15 |
| Human IgG1 | NA |

### 4.2 Affinity measurement of CD47 antibody

The binding affinity between the antigen and the antibody was measured using a BIAcore T200 instrument. The binding strength between the CD47 antigen and the antibody was determined using a CM5 chip (purchased from Cytiva, Cat# 29149603) via amine coupling. First, the CD47 antigen was immobilized onto the CM5 chip. According to the instructions of the Amine Coupling Kit (purchased from Cytiva, Cat# BR100050), using PBST as the running buffer, NHS and EDC were mixed and injected to activate the chip for approximately 420 seconds. The CD47 antigen was diluted to 10 µg/mL in 10 mM sodium acetate (pH 5.5) and injected to achieve an immobilization level of 141.2 RU. Finally, the remaining activated sites were blocked with 1 M ethanolamine (pH 8.5). Subsequently, the affinity of the antibody for the CD47 antigen was determined using the multi-cycle kinetics method. In each cycle, a single concentration of the antibody protein was injected, and the association and dissociation processes between the antibody and the antigen protein were recorded. Finally, the chip was regenerated with Glycine-HCl (pH 1.5). The running buffer was PBST, the flow rate was 30 µL/min, the regeneration time was 15 s, and the detection temperature was 25 °C. Data were analyzed according to a 1:1 binding model, and the kinetic parameters of antibody-antigen binding, including the association rate constant (Ka), dissociation rate constant (Kd), equilibrium dissociation constant (KD), and maximum binding signal (Rmax), were fitted.

The association rate (Ka), dissociation rate (Kd), and binding affinity (KD) of the CD47 antibodies for the human CD47 antigen are shown in Table 2.2. Compared to 70H1, the affinity of the CD47 TMEAbody was decreased by 470-fold.

**Table 2.2 Affinity of CD47 antibodies for human CD47 antigen as determined by SPR (Biacore)**

| Antibody name | Ka (1/Ms) | Kd (1/s) | KD (M) |
|---|---|---|---|
| 70H1 | 4.54E+06 | 1.40E-04 | 3.08E-11 |
| CD47 TMEAbody | 2.44E+04 | 3.43E-04 | 1.41E-08 |

### 4.3 The binding of CD47 TMEAbody to CHOK1-CD47 cells is reduced and can be restored

The desired overexpressing cell line, CHOK1-CD47 (cultured in RPMI-1640 (ATCC Modification, Gibco, Cat# A10491-01) + 10% FBSThe (BI, Cat# 04-001-1A) + 1× P/S + 6 µg/ml Puromycin (Gibco, Cat# A1113803, 10 × 1 ml (10 mg/ml))), was expanded to the logarithmic phase. Cells were digested, collected, and pipetted to create a single-cell suspension. Following cell counting, the suspension was centrifuged, and the supernatant was discarded. The cells were resuspended in FACS buffer (PBS + 2% FBS) to a density of 2×10⁶/ml, and added to a 96-well plate (Corning, Cat# 3799) with 100 µl/well. After centrifugation and removal of the supernatant, 100 µl/well of the antibody sample to be tested (initial concentration of 100 nM, 5-fold gradient dilutions, 8 dilution points) was added, mixed with the cells, and incubated at 4 °C for 1 hour. Cells were washed by centrifugation with FACS buffer for three times, then added with 100 µl/well of Goat anti-Human IgG Fc Secondary Antibody, PE (eBioscience^{™}, Invitrogen, Cat# 12-4998-82, 1:200 dilution) and incubated at 4 °C for 1 hour. After three washes with FACS buffer by centrifugation, the cells were resuspended in 200 µl/well of FACS buffer and analyzed using FACS (BECKMAN COULTER, CytoFLEX). Data were fitted and the results were analyzed using software (GraphPad Prism 8).

As shown in Figure 4 and Table 3, the binding of the 70H1 TMEAbody to cells was approximately 20-fold lower compared to 70H1. However, when being activated with asparaginyl endopeptidase, the activated 70H1 TMEAbody restored binding comparable to that of 70H1 IgG1-mutation.

Therefore, the CD47 TMEAbody (70H1. IgG1 + 20K PEG) was shown to largely block the binding ability of the 70H1 antibody to CHOK1-CD47 cells. At the same time, the activated CD47 TMEAbody was able to restore its binding ability to CHOK1-CD47 cells.

**Table 3**

| Antibody name | EC50 (nM) |
|---|---|
| 70H1.IgG1 | 0.38 |
| CD47 TMEAbody | 7.6 |
| CD47 TMEAbody activated | 0.91 |
| 70H1.IgG1.Y61C | 0.39 |

### Example 5. CD47 TMEAbody attenuates the blocking functional activity of CD47 antibody and can be restored

### 5.1 Dose-dependent binding of CD47 antibody to Raji cells

The required cell line, Raji (cultured in RPMI-1640 (ATCC Modification, Gibco, Cat# A10491-01) + 10% FBS (BI, Cat# 04-001-1A) + 1× P/S), was expanded to the logarithmic phase. Cells were collected and pipetted to create a single-cell suspension, followed by centrifugation and removal of the supernatant. The cells were resuspended in FACS buffer (PBS + 2% FBS) and counted, and the cell density was adjusted to 2×10⁶/ml. 100 µl/well of this suspension was added to a 96-well plate (Corning, Cat# 3799). After centrifugation and removal of the supernatant, 100 µl/well of the antibody sample to be tested (initial concentration of 100 nM, 5-fold gradient dilutions) was added, mixed with the cells, and incubated at 4 °C for 1 hour. Cells were washed by centrifugation with FACS buffer for three times, then added with 100 µl/well of Goat anti-Human IgG Fc Secondary Antibody, PE (eBioscience^{™}, Invitrogen, Cat# 12-4998-82, 1:200 dilution) and incubated at 4 °C for 1 hour. After three washes with FACS buffer by centrifugation, the cells were resuspended in 200 µl/well of FACS buffer and analyzed using FACS (BECKMAN COULTER, CytoFLEX). Data were fitted and the results were analyzed using software (GraphPad Prism 8).

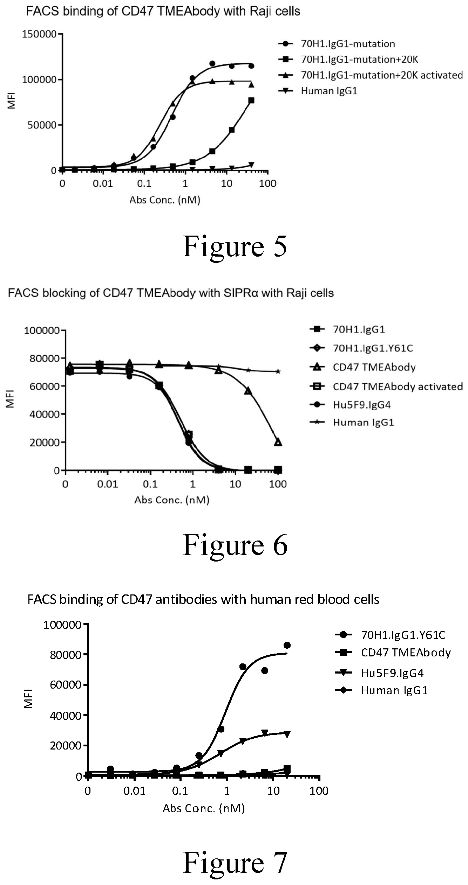

As shown in Figure 5 and Table 4, the binding of the CD47 TMEAbody to Raji cells was approximately 60-fold lower compared to 70H1. However, when being activated with asparaginyl endopeptidase, the activated 70H1 TMEAbody restored binding comparable to that of 70H1 IgG1-mutation.

**Table 4**

| Antibody name | EC50 (nM) |
|---|---|
| 70H1.IgG1.Y61C | 0.47 |
| CD47 TMEAbody | 31.94 |
| CD47 TMEAbody activated | 0.24 |
| Human IgG1 | NA |

### 5.2 Detection of the blocking functional activity of CD47 antibodies

The required cell line, Raji (cultured in RPMI-1640 (ATCC Modification, Gibco, Cat# A10491-01) + 10% FBS (BI, Cat# 04-001-1A) + 1× P/S), was expanded to the logarithmic phase. Cells were collected and pipetted to create a single-cell suspension, followed by centrifugation and removal of the supernatant. The cells were resuspended in FACS buffer (PBS + 2% FBS) and counted, and the cell density was adjusted to 2×10⁶/ml. 100 µl/well of this suspension was added to a 96-well plate (Corning, Cat# 3799). After centrifugation and removal of the supernatant, 100 µl/well of the antibody sample to be tested (CD47 Antibodies at an initial concentration of 200 nM, with 5-fold gradient dilutions and 8 dilution points, mixed with an equal volume of Human SIRP alpha, mFc (DIMA Biotech, Cat# PME100531) at 16 µg/ml) was added, mixed with the cells, and incubated at 4 °C for 1 hour. Cells were washed by centrifugation with FACS buffer for three times, then added with 100 µl/well of Goat F(ab')2 Anti-Mouse IgG-Fc (PE), pre-adsorbed (abcam, Cat# ab98742, 1:200 dilution) and incubated at 4 °C for 1 hour. After three washes with FACS buffer by centrifugation, the cells were resuspended in 200 µl/well of FACS buffer and analyzed using FACS (BECKMAN COULTER, CytoFLEX). Data were fitted and the results were analyzed using software (GraphPad Prism 8).

As shown in Figure 6, the activity of the CD47 TMEAbody (70H1. IgG1-mutation + 20K PEG) in blocking the binding of CD47 on Raji cells to SIRPα was significantly reduced. This blocking activity was restored after the CD47 TMEAbody was activated.

### Example 6. Blocking of CD47 antibody binding to red blood cells by CD47 TMEAbody

Human red blood cells (RBCs) (XFB-RBC, 10 ml) were centrifuged, and the supernatant was discarded. The cells were resuspended in FACS buffer (PBS + 2% FBS) and counted, and the cell density was adjusted to 2×10⁶/ml. 100 µl/well of this suspension was added to a 96-well plate. After centrifugation and removal of the supernatant, the antibody to be tested (initial concentration of 100 nM, 5-fold gradient dilutions, 8 dilution points) was added at 100 µl/well, mixed with the cells, and incubated at 4 °C for 1 hour. Cells were washed by centrifugation with FACS buffer for three times, then added with 100 µl/well of Goat anti-Human IgG Fc Secondary Antibody, PE (eBioscience^{™}, Invitrogen, Cat# 12-4998-82, 1:200 dilution) and incubated at 4 °C for 1 hour. After three washes with FACS buffer by centrifugation, the cells were resuspended in 200 µl/well of FACS buffer and analyzed using FACS (BECKMAN COULTER, CytoFLEX). Data were fitted and the results were analyzed using software (GraphPad Prism 8).

As shown in Figure 7, the CD47 antibody exhibited relatively strong binding to red blood cells, whereas the CD47 TMEAbody showed essentially no binding to red blood cells.

### Example 7. Human red blood cell (RBC) agglutination assay

Human red blood cells (RBCs) (XFB-RBC, 10 ml) were centrifuged, and the supernatant was discarded. The cells were washed twice with wash buffer (1×PBS + 0.05 M EDTA), then the cells were resuspended in PBS and counted, and the cell density was adjusted to 2×10⁶/ml. 100 µl/well of this suspension was added to a 96-well plate (Corning, Cat# 3799), centrifuged, and the supernatant was discarded. 50 µl/well of the antibody to be tested (initial concentration of 100 nM, 5-fold gradient dilutions, 7 points + 0 point) was added and incubated at 37 °C for 1 hour.

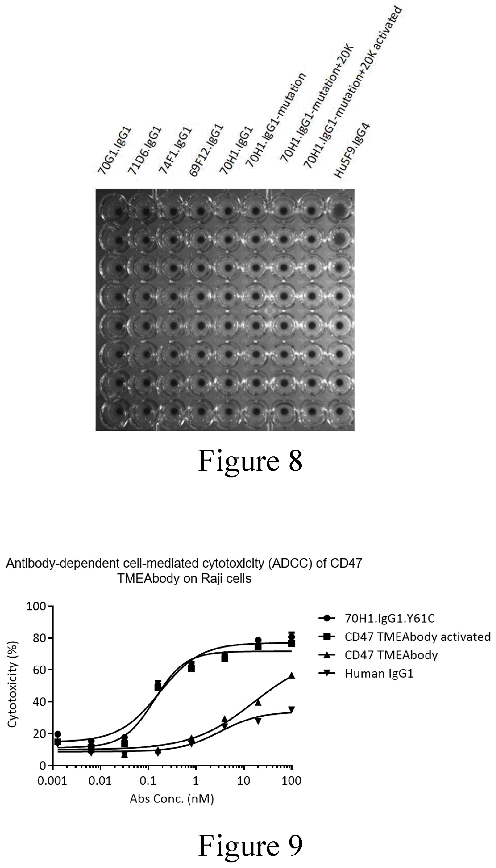

As shown in Figure 8, except for the positive antibody 5F9, which caused red blood cell agglutination at high concentrations, none of the other CD47 antibodies caused red blood cell agglutination.

### Example 8. CD47 TMEAbody losses antibody-dependent cell-mediated cytotoxicity (ADCC) activity and this activity can be restored

Raji cells (cultured in RPMI-1640 (ATCC Modification, Gibco, Cat# A10491-01) + 10% FBS (BI, Cat# 04-001-1A) + 1× P/S) were expanded to the logarithmic phase. Cells were collected and pipetted to create a single-cell suspension. After centrifugation, the supernatant was discarded, and the cells were resuspended in culture medium, counted, and the cell density was adjusted to 2×10⁵/ml. 50 µl/well of this suspension was added to a 96-well cell culture plate (Corning, Cat# 3599) and incubated overnight at 37°C in a 5% CO₂ atmosphere. PBMCs (XF, Cat# XC11100W) were resuspended in 10% FBS-RPMI1640 medium. After centrifugation, the supernatant was discarded, and the cell density was adjusted to 8×10⁶/ml. 50 µl/well of the antibody to be tested and 50 µl of the PBMC suspension (E:T ratio of 20:1) were added to the culture plate. The plate was placed in a CO₂ incubator and incubated for 24 hours. The ADCC assay was performed using an LDH detection kit (Cytotoxicity Detection KitPLUS (LDH), Roche, Cat# 4744934001).

As shown in Figure 9 and Table 5, compared to the 70H1 antibody, the CD47 TMEAbody lost its ADCC activity. This ADCC activity was restored after the CD47 TMEAbody was activated.

**Table 5**

| Antibody name | EC50 (nM) |
|---|---|
| 70H1.IgG1.Y61C | 0.17 |
| CD47 TMEAbody | 16.32 |
| CD47 TMEAbody activated | 0.14 |
| Human IgG1 | NA |

### Example 9. Humanization of 70H1

The humanized light chain template for the rabbit-derived antibody 70H1 is IGKV1_5, and the humanized heavy chain template is IGHV3_NL1. The CDRs of the rabbit-derived antibody 70H1 were grafted into the corresponding human templates. Key amino acids in the FR regions of the antibody were then back-mutated to ensure the original affinity maintained. The specific mutation designs are shown in Table 6. Finally, the different heavy chains and light chains obtained from the backmutation of the humanized antibodies were combined to generate 12 humanized antibodies.

**Table 6.**

| | |
|---|---|
| VH1 SEQ. 11 | QVQLVESGGGVVQPGGSLRLSCAASGFTFSSGYDMCWVRQAPGKGLEW VSCIYTRSSGGTYYASWAKGRFTISRDNSKNTLYLQMNSLRAEDTAVYYC AKGRSSYRLNLWGQGTLVTVSS |
| VH2 SEQ. 12 | QQQLVESGGGVVQPGGSLRLSCAASGFTFSSGYDMCWVRQAPGKGLEWI ACIYTRSSGGTYYASWAKGRFTISKDNSKNTLYLQMNSLRAEDTAVYYCA KGRSSYRLNLWGQGTLVTVSS |
| VH3 SEQ. 13 | QQQLVESGGGVVQPGGSLRLSCAASGFSFSSGYDMCWVRQAPGKGLEWI ACIYTRSSGGTYYASWAKGRFTISKDNSKNTLYLQMNSLRAEDTAVYFCA KGRSSYRLNLWGQGTLVTVSS |
| VH4 SEQ. 14 | QQQLVESGGGVVQPGGSLRLSCAASGFSFSSGYDMCWVRQAPGKGLEWI ACIYTRSSGGTYYASWAKGRFTISKDNSKNTVYLQMNSLRAEDTAVYFCA KGRSSYRLNLWGQGTLVTVSS |
| VL1 SEQ. 15 | DIQMTQSPSTLSASVGDRVTITCQASQSISNELSWYQQKPGKAPKLLIYRA STLESGVPSRFSGSGSGTEFTLTISSLQPDDFATYYCQSTFYGDYGNAFGGG TKVEIK |
| VL2 SEQ. 16 | DIQMTQSPSTLSASVGDRVTIKCQASQSISNELSWYQQKPGKAPKLLIYRA STLESGVSSRFSGSGSGTEFTLTISSLQPDDFATYYCQSTFYGDYGNAFGGG TKVEIK |
| VL3 SEQ. 17 | DIQMTQSPSTLSASVGDRVTIKCQASQSISNELSWYQQKPGKPPKLLIYRA STLESGVSSRFSGSGSGTEFTLTISSLQPDDFATYYCQSTFYGDYGNAFGGG TKVEIK |

| | |
|---|---|
| SEQ. represents SEQ ID NO.. | |

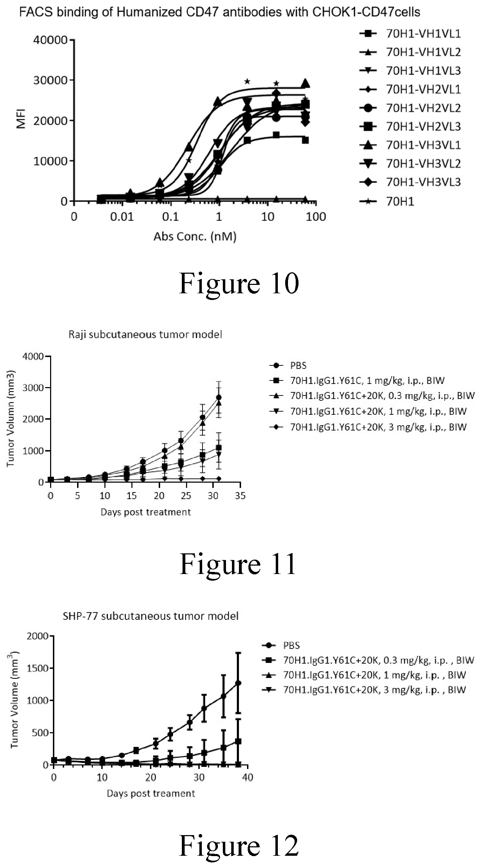

FACS binding of the 12 different humanized antibodies to CHOK1-CD47 cells is shown in Figure 10. The FACS EC50 values of the different antibodies are shown in Table 7. The final preferred antibody is 70H1-VH3VL1.

**Table 7**

| Antibody name | EC50 (nM) |
|---|---|
| 70H1-VH1VL1 | 1.04 |
| 70H1-VH1VL2 | NA |
| 70H1-VH1VL3 | 1.76 |
| 70H1-VH2VL1 | 1.09 |
| 70H1-VH2VL2 | 1.17 |
| 70H1-VH2VL3 | 1.05 |
| 70H1-VH3VL1 | 0.23 |
| 70H1-VH3VL2 | 0.64 |
| 70H1-VH3VL3 | 1.01 |
| 70H1 | 0.35 |

### Example 10. In vivo pharmacological efficacy of CD47 TMEAbody in a mouse tumor (RAJI) model

Human Burkitt's lymphoma RAJI cells were subcutaneously transplanted into NOD-SCID mice to establish an *in vivo* model. RAJI cells were cultured according to standard culture conditions and were collected during the exponential growth phase for counting. Each mouse was inoculated on the right flank with 1×10⁷ human Burkitt's lymphoma RAJI cells (suspended in 0.1 ml of basal medium). After inoculation, when the tumors grew to 80-114 mm³ (7 days post-tumor implantation), the mice were randomly grouped based on body weight and tumor volume. The experimental groups were divided as follows:

### PBS group,

70H1.IgG1.Y61C (1 mg/kg) group,
70H1.IgG1.Y61C+20K (0.3 mg/kg) group,
70H1.IgG1.Y61C+20K (1 mg/kg) group,
70H1.IgG1.Y61C+20K (3 mg/kg) group.

Here, 70H1.IgG1.Y61C+20K refers to the CD47 TMEAbody conjugated with PEG of approximately 20K molecular weight.

Treatment was started on the day of grouping. Mice were weighed, and tumor volumes were measured twice a week. Tumor volume was calculated using the formula: tumor volume = 1/2 × length × width × width (mm³). The tumor growth inhibition rate TGI (%) was calculated as [1 - (Tt - T0) / (Ct - C0)] × 100%. The tumor inhibition rate T/C (%) was calculated as (Tt/T0) / (Ct/C0) × 100%. T0 represents the tumor volume of the test antibody group at the time of grouping; Tt represents the tumor volume of the test antibody group at each measurement; C0 represents the average tumor volume of the PBS group at the time of grouping; Ct represents the average tumor volume of the PBS group at each measurement.

As shown in Figure 11, 70H1.IgG1.Y61C+20K demonstrated significant tumor growth inhibition in the subcutaneous RAJI transplanted tumor model in mice. After 31 days of treatment, the TGI inhibition rates of the 70H1.IgG1.Y61C (1 mg/kg) group, the 70H1.IgG1.Y61C+20K (0.3 mg/kg) group, the 70H1.IgG1.Y61C+20K (1 mg/kg) group, the 70H1.IgG1.Y61C+20K (3 mg/kg) group, and the 5F9 (1 mg/kg) group were 60.91%, 6.45%, 69.46%, 98.97%, and 74.55%, respectively. The T/C ratios were 41.81%, 93.73%, 33.06%, 4.51%, and 28.36%, respectively. No significant changes in body weight were observed during the treatment period.

As shown in Figure 11, the results indicated that 70H1.IgG1.Y61C+20K could be activated in the tumor microenvironment and stimulate an anti-tumor immune response. It demonstrated similar *in vivo* efficacy to 70H1.IgG1.Y61C while allowing for an increased administration dose. The specific tumor volume data at different days (Day, abbreviated as D) are shown in Table 8.1 and Table 8.2 below.

**Table 8.1. Specific Tumor Volume Data at Different Days**

| Tumor Volume (mm³) | D0 | D3 | D7 | D10 | D14 |
|---|---|---|---|---|---|
| PBS | 94.52 | 118.51 | 174.92 | 257.35 | 439.54 |
| 70H1.IgG1.Y61C (1 mg/kg) | 93.05 | 101.19 | 109.48 | 154.21 | 244.09 |
| 70H1.IgG1.Y61C+2 0K (0.3 mg/kg) | 94.56 | 123.83 | 147.44 | 232.04 | 358.82 |
| 70H1.IgG1.Y61C+2 0K (1 mg/kg) | 94.25 | 100.17 | 116.36 | 162.08 | 214.12 |
| 70H1.IgG1.Y61C+2 0K (3 mg/kg) | 93.15 | 91.90 | 81.07 | 87.74 | 92.16 |

**Table 8.2. Specific Tumor Volume Data at Different Days**

| Tumor Volume (mm³) | D17 | D21 | D24 | D28 | D31 |
|---|---|---|---|---|---|
| PBS | 662.35 | 1022.72 | 1332.80 | 2069.66 | 2701.05 |
| 70H1.IgG1.Y61C (1 mg/kg) | 358.92 | 525.12 | 640.85 | 889.73 | 1111.84 |
| 70H1.IgG1.Y61C+ 20K (0.3 mg/kg) | 511.34 | 849.95 | 1156.48 | 1887.63 | 2532.90 |
| 70H1.IgG1.Y61C+ 20K (1 mg/kg) | 307.27 | 384.91 | 485.51 | 679.76 | 890.40 |
| 70H1.IgG1.Y61C+ 20K (3 mg/kg) | 91.91 | 129.68 | 117.69 | 125.24 | 120.05 |

### Example 11. In vivo pharmacological efficacy of CD47 TMEAbody in a mouse tumor (SHP-77) model

Human small cell lung cancer SHP-77 cells were subcutaneously transplanted into CB17-SCID mice to establish an *in vivo* model. SHP-77 cells were cultured according to standard culture conditions and were collected during the exponential growth phase for counting. Each mouse was inoculated on the right flank with 2.5×10⁶ human small cell lung cancer SHP-77 cells (suspended in 0.1 ml of basal medium). After inoculation, when the tumors grew to 60-103 mm³ (5 days post-tumor implantation), the mice were randomly grouped based on body weight and tumor volume. The experimental groups were divided as follows:

### PBS group,

70H1.IgG1.Y61C+20K (0.3 mg/kg) group,
70H1.IgG1.Y61C+20K (1 mg/kg) group,
70H1.IgG1.Y61C+20K (3 mg/kg) group.

Here, 70H1.IgG1.Y61C+20K refers to the CD47 TMEAbody conjugated with PEG of approximately 20K molecular weight (i.e., PEG20K).

Treatment was started on the day of grouping. Mice were weighed, and tumor volumes were measured twice a week. Tumor volume was calculated using the formula: tumor volume = 1/2 × length × width × width (mm³). The tumor growth inhibition rate TGI (%) was calculated as [1 - (Tt - T0) / (Ct - C0)] × 100%. The tumor inhibition rate T/C (%) was calculated as (Tt/T0) / (Ct/C0) × 100%. T0 represents the tumor volume of the test antibody group at the time of grouping; Tt represents the tumor volume of the test antibody group at each measurement; C0 represents the average tumor volume of the PBS group at the time of grouping; Ct represents the average tumor volume of the PBS group at each measurement.

As shown in Figure 12, 70H1.IgG1.Y61C+20K demonstrated significant tumor growth inhibition in the subcutaneous SHP77 transplanted tumor model in mice. After 38 days of treatment, the TGI inhibition rates of the 70H1.IgG1.Y61C+20K (0.3 mg/kg) group, the 70H1.IgG1.Y61C+20K (1 mg/kg) group, and the 70H1.IgG1.Y61C+20K (3 mg/kg) group were 75.38%, 105.46%, and 105.53%, respectively. The T/C ratios were 29.52%, 1.13%, and 1.02%, respectively. No significant changes in body weight were observed during the treatment period.

As shown in Figure 12, the results indicated that 70H1.IgG1.Y61C+20K could be activated in the tumor microenvironment and stimulate an anti-tumor immune response. The specific tumor volume data at different days are shown in Table 9.1 and Table 9.2 below.

**Table 9.1. Specific Tumor Volume Data at Different Days**

| Tumor Volume (mm³) | D0 | D3 | D7 | D10 | D14 | D17 |
|---|---|---|---|---|---|---|
| PBS | 79.79 | 102.80 | 94.22 | 100.24 | 154.06 | 229.31 |
| 70H1.IgG1.Y61C+20K (0.3 mg/kg) | 79.15 | 63.51 | 44.29 | 42.63 | 42.49 | 44.81 |
| 70H1.IgG1.Y61C+20K (1 mg/kg) | 79.75 | 69.70 | 47.94 | 36.61 | 32.95 | 20.93 |
| 70H1.IgG1.Y61C+20K (3 mg/kg) | 79.00 | 65.86 | 44.80 | 33.94 | 27.48 | 24.62 |

**Table 9.2. specific tumor volume data at different days**

| Tumor Volume (mm³) | D21 | D24 | D28 | D31 | D35 | D38 |
|---|---|---|---|---|---|---|
| PBS | 335.83 | 480.81 | 666.30 | 883.83 | 1071.56 | 1275.92 |
| 70H1.IgG1.Y61C+20K (0.3 mg/kg) | 74.08 | 116.17 | 142.04 | 194.29 | 272.39 | 373.67 |
| 70H1.IgG1.Y61C+20K (1 mg/kg) | 19.98 | 27.75 | 17.69 | 21.42 | 15.37 | 14.44 |
| 70H1.IgG1.Y61C+20K (3 mg/kg) | 9.14 | 13.52 | 10.79 | 17.13 | 14.44 | 12.85 |

### Example 12. In vivo pharmacological efficacy of CD47 TMEAbody in a mouse tumor (SHP-77) model

Human small cell lung cancer SHP-77 cells were subcutaneously transplanted into CB17-SCID mice to establish an *in vivo* model. SHP-77 cells were cultured according to standard culture conditions and were collected during the exponential growth phase for counting. Each mouse was inoculated on the right flank with 2.5×10⁶ human small cell lung cancer SHP-77 cells (suspended in 0.1 ml of basal medium). After inoculation, when the tumors grew to 60-140 mm³ (18 days post-tumor implantation), the mice were randomly grouped based on body weight and tumor volume. The experimental groups were divided as follows:

### PBS group,

70H1.IgG1.Y61C+40K (3 mg/kg) group,
70H1.IgG1.Y61C+40K (6 mg/kg) group,
70H1.IgG1.Y61C+40K (9 mg/kg) group.

Here, 70H1.IgG1.Y61C+40K refers to the CD47 TMEAbody conjugated with PEG of approximately 40K molecular weight (i.e., PEG40K).

Treatment was started on the day of grouping. Mice were weighed, and tumor volumes were measured twice a week. Tumor volume was calculated using the formula: tumor volume = 1/2 × length × width × width (mm³). The tumor growth inhibition rate TGI (%) was calculated as [1 - (Tt - T0) / (Ct - C0)] × 100%. The tumor inhibition rate T/C (%) was calculated as (Tt/T0) / (Ct/C0) × 100%. T0 represents the tumor volume of the test antibody group at the time of grouping; Tt represents the tumor volume of the test antibody group at each measurement; C0 represents the average tumor volume of the PBS group at the time of grouping; Ct represents the average tumor volume of the PBS group at each measurement.

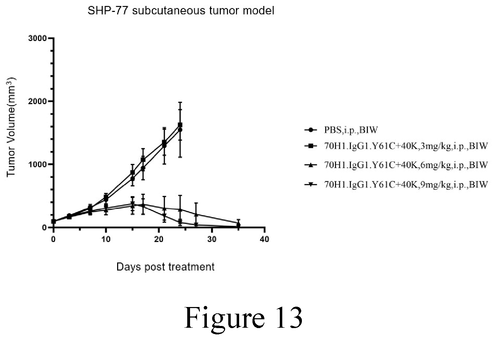

As shown in Figure 13, 70H1.IgG1.Y61C+40K demonstrated significant tumor growth inhibition in the subcutaneous SHP77 transplanted tumor model in mice. After 24 days of treatment, the TGI inhibition rates of the 70H1.IgG1.Y61C+40K (3 mg/kg) group, the 70H1.IgG1.Y61C+40K (6 mg/kg) group, and the 70H1.IgG1.Y61C+40K (9 mg/kg) group were - 5.23%, 86.99%, and 101.20%, respectively. The T/C ratios were 105.11%, 18.43%, and 5.12%, respectively. No significant changes in body weight were observed during the treatment period.

As shown in Figure 13, the results indicated that 70H1.IgG1.Y61C+40K could be activated in the tumor microenvironment and stimulate an anti-tumor immune response. The specific tumor volume data at different days are shown in Table 10.1 and Table 10.2 below.

**Table 10.1. Specific Tumor Volume Data at Different Days**

| Tumor Volume (mm³) | D0 | D3 | D7 | D10 | D15 |
|---|---|---|---|---|---|
| PBS | 96.83 | 189.99 | 317.51 | 440.44 | 775.93 |
| 70H1.IgG1.Y61C+40K (3 mg/kg) | 96.63 | 170.21 | 303.33 | 484.87 | 872.23 |
| 70H1.IgG1.Y61C+40K (6 mg/kg) | 96.90 | 166.35 | 243.57 | 275.20 | 343.56 |
| 70H1.IgG1.Y61C+40K (9 mg/kg) | 96.76 | 176.95 | 259.90 | 306.67 | 376.09 |

**Table 10.2. specific tumor volume data at different days**

| Tumor Volume (mm³) | D17 | D21 | D24 | D27 | D35 |
|---|---|---|---|---|---|
| PBS | 943.78 | 1294.13 | 1549.90 | - | - |
| 70H1.IgG1.Y61C+40K (3 mg/kg) | 1073.58 | 1352.02 | 1625.70 | - | - |
| 70H1.IgG1.Y61C+40K (6 mg/kg) | 368.63 | 303.25 | 285.90 | 207.99 | 69.52 |
| 70H1.IgG1.Y61C+40K (9 mg/kg) | 330.31 | 183.09 | 79.28 | 41.09 | 13.50 |

### Example 13. In vivo toxicity characterization in hSIRPA/hCD47 (C57 background) mice

hSIRPA/hCD47 (C57 background) mice were randomly divided into 4 groups based on body weight, with 3 mice per group. The hematological toxicity of 70H1.IgG1.Y61C+20K and 70H1.IgG1.Y61C+40K was studied via intraperitoneal injection.

The experimental groups were divided as follows:
70H1.IgG1.Y61C+20K (5 mg/kg) group,
70H1.IgG1.Y61C+40K (10 mg/kg) group,
70H1.IgG1.Y61C+40K (20 mg/kg) group,
70H1.IgG1.Y61C+40K (30 mg/kg) group.

After grouping, a single dose of the corresponding antibody was administered intraperitoneally to the mice. Blood cell counting was performed on the mice at D0 (pre-dose), D2, D5, D7, D9, and D12. Changes in red blood cells and platelets were observed to assess the effect of the antibodies on the mouse hematology.

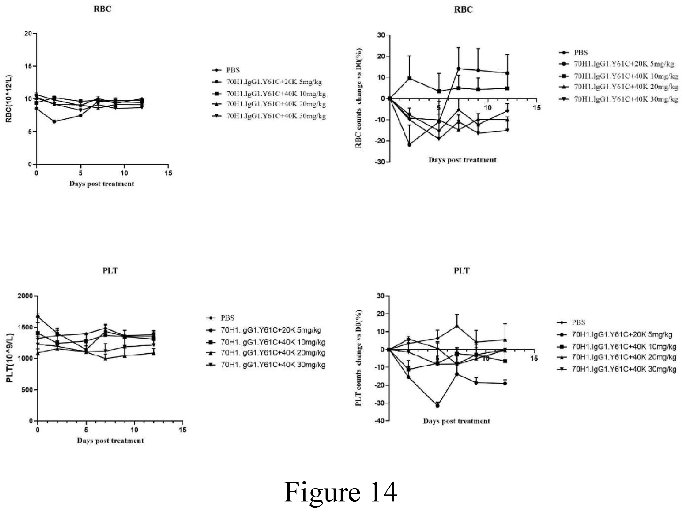

As shown in Figure 14: For 70H1.IgG1.Y61C+20K at 5 mg/kg, a reversible decrease in red blood cells was observed on D2 post-administration. For the 70H1.IgG1.Y61C+40K groups, a minor decrease in red blood cells was observed, with no significant difference compared to the PBS group across all dose levels. For 70H1.IgG1.Y61C+20K at 5 mg/kg, a reversible decrease in platelets was observed on D5 post-administration. For the 70H1.IgG1.Y61C+40K groups, no significant decrease in platelets was observed.

The specific data for mouse red blood cells and platelets at different days are shown in Tables 11.1 and 11.2.

**Table 11.1. Specific data for mouse red blood cells (RBC) at different days (unit: 10^12/L):**

| Group | RBC Count | D0 | D2 | D5 | D7 | D9 | D12 |
|---|---|---|---|---|---|---|---|
| PBS | Mean | 10.65 | 9.83 | 9.01 | 10.06 | 9.33 | 10.02 |
| | Change Rate (%) | | -0.08 | -0.15 | -0.06 | -0.12 | -0.06 |
| 70H1.IgG1.Y61C+ 20K 5mg/kg | Mean | 8.53 | 6.56 | 7.48 | 9.62 | 9.55 | 9.45 |
| | Change Rate (%) | | -0.23 | -0.12 | 0.13 | 0.12 | 0.11 |
| 70H1.IgG1.Y61C+ 40K 10mg/kg | Mean | 9.42 | 10.18 | 9.63 | 9.79 | 9.76 | 9.78 |
| | Change Rate (%) | | 0.08 | 0.02 | 0.04 | 0.04 | 0.04 |
| 70H1.IgG1.Y61C+ | Mean | 10.12 | 9.19 | 9.09 | 8.62 | 9.14 | 9.12 |
| 40K 20mg/kg | Change Rate (%) | | -0.09 | -0.10 | -0.15 | -0.10 | -0.10 |
| 70H1.IgG1.Y61C+ 40K 30mg/kg | Mean | 10.25 | 9.20 | 8.27 | 9.12 | 8.56 | 8.67 |
| | Change Rate (%) | | -0.10 | -0.19 | -0.11 | -0.17 | -0.15 |

| | | | | | | | |
|---|---|---|---|---|---|---|---|
| Note: The change rate (%) refers to the percentage change in mouse red blood cells on different days compared to D0 (pre-dose). | | | | | | | |

**Table 11.2. Specific data for mouse platelets (PLT) at different days (unit: 10^9/L):**

| Group | PLT Count | D0 | D2 | D5 | D7 | D9 | D12 |
|---|---|---|---|---|---|---|---|
| PBS | Mean | 1320.33 | 1367.00 | 1397.67 | 1487.33 | 1367.33 | 1380.33 |
| | Change Rate (%) | | 0.04 | 0.06 | 0.13 | 0.04 | 0.05 |
| 70H1.IgG1.Y61C +20K 5mg/kg | Mean | 1668.33 | 1406.33 | 1142.33 | 1435.33 | 1356.00 | 1351.00 |
| | Change Rate (%) | | -0.16 | -0.32 | -0.14 | -0.19 | -0.19 |
| 70H1.IgG1.Y61C +40K 10mg/kg | Mean | 1408.67 | 1244.33 | 1278.67 | 1374.00 | 1348.00 | 1310.00 |
| | Change Rate (%) | | -0.12 | -0.09 | -0.02 | -0.04 | -0.07 |
| 70H1.IgG1.Y61C +40K 20mg/kg | Mean | 1090.67 | 1155.67 | 1102.67 | 998.33 | 1041.33 | 1087.67 |
| | Change Rate (%) | | 0.06 | 0.01 | -0.08 | -0.05 | 0.00 |
| 70H1.IgG1.Y61C +40K 30mg/kg | Mean | 1225.00 | 1195.67 | 1108.00 | 1114.33 | 1186.33 | 1215.67 |
| | Change Rate (%) | | -0.02 | -0.10 | -0.09 | -0.03 | -0.01 |

| | | | | | | | |
|---|---|---|---|---|---|---|---|
| Note: The change rate (%) refers to the percentage change in mouse platelets on different days compared to D0 (pre-dose). | | | | | | | |

The sequences involved in the present invention are as follows:
SEQ ID NO. 1 70G1 VH 
QSVKESEGGLFKPTDTLTLTCTVSGFSLSNYAINWVRQAPGNGLEWIGIINGYGMTY
YASWAKSRSTITRNTSENTVTLKLTSLTAADTATYFCARDRYGSYVGYSSDIWGPGTLVT
 VSS
SEQ ID NO. 2 70G1 VL 
AYDMTQTPASVEAAVGGTVTIKCQASQNISSRLAWYQQKPGQPPKLLIYGTSTLASG
VPSRFRGSGSGTQFSLTISDLECADAATYYCQHGYYYSSSFTTYNAFGGGTEVVVK
 SEQ ID NO. 3 71D6 VH 
QSLEESGGDLVKPGASLTLTCTASGFSFSTSYWIYWVRQAPGKGLELIACIATGSDGS
TYYASWASGRFTISKTSSTTVTLQMTSLTAADTATYFCAREAGNGVYNLWGQGTLVTVS
S
SEQ ID NO. 4 71D6 VL 
AYDMTQTPASVEVAVGGTVTIKCQASESIRNALAWYQQKPGQPPKLLISGASKLASG
VASRFKGSGSATEFTLTISDLECADAATYYCQQYYSSSNVDNGFGGGTEVVVK
 SEQ ID NO. 5 74F1 VH 
QQQLEESGGDLVKPEGSLTLTCTASGFSFSSRYNMCWVRQAPGKGLEWIACIYTGSS
GSTYYASWAKGRFTISKTSSTVDLKMTSLTAADTATYFCAGGSYSYRLNLWGQGTLVTV
SS
SEQ ID NO. 6 74F1 VL 
DVVMTQTPASVSTAVGGTVTIKCQASQSISNELSWYQQKPGQPPKLLIYRASTLESG
VSSRFSGSGSGTEFTLTISDLECADAATYYCQATGYGDFGNAFGGGTEVVVK
 SEQ ID NO. 7 70H1 VH 
QQQLEESGGGLVKPEGSLTLTCTASGFSFSSGYDMCWVRQAPGRGLEWIACIYTRSS
GGTYYASWAKGRFTISKTSSTVDLRMTSLTAADTATYFCAGGRSSYRLNLWGQGALVTV
SS
SEQ ID NO. 8 70H1 VL 
DVVMTQTPSPVEAAVGGTVTIKCQASQSISNELSWYQQKPGQPPKLLIYRASTLESG
VSSRFKGSGSGTEFTLTISDLECADAATYYCQSTFYGDYGNAFGGGTEVVVK
 SEQ ID NO. 9 69F12 VH 
QSVEESEGGLFKPTDTLTLTCTISGFSLSNYDMIWVRQAPGNGLEYIGIISSRDKAGH
AAWAKRRSTITRNPKENTVTLKMTSLTVADTATYFCARRFSSMHGLDIWGPGTLVTVSS
SEQ ID NO. 10 69F12 VL 
AYDMTQTPSSVSAAVGGTVTIKCQASQSISNYLSWYQQKPGQPPKLLIYLASTLASG
VPSRFKGSGSGTQFTLTISGVQCADAATYYCQQGYSSDSVDNVFGGGTEVVVK
SEQ ID NO. 11 70H1 VH1 
QVQLVESGGGVVQPGGSLRLSCAASGFTFSSGYDMCWVRQAPGKGLEWVSCIYTR
SSGGTYYASWAKGRFTISRDNSKNTLYLQMNSLRAEDTAVYYCAKGRSSYRLNLWGQG
TLVTVSS
SEQ ID NO. 12 70H1 VH2 
QQQLVESGGGVVQPGGSLRLSCAASGFTFSSGYDMCWVRQAPGKGLEWIACIYTRS
SGGTYYASWAKGRFTISKDNSKNTLYLQMNSLRAEDTAVYYCAKGRSSYRLNLWGQGT
LVTVSS
SEQ ID NO. 13 70H1 VH3 
QQQLVESGGGVVQPGGSLRLSCAASGFSFSSGYDMCWVRQAPGKGLEWIACIYTRS
SGGTYYASWAKGRFTISKDNSKNTLYLQMNSLRAEDTAVYFCAKGRSSYRLNLWGQGT
LVTVSS
SEQ ID NO. 14 70H1 VH4 
QQQLVESGGGVVQPGGSLRLSCAASGFSFSSGYDMCWVRQAPGKGLEWIACIYTRS
SGGTYYASWAKGRFTISKDNSKNTVYLQMNSLRAEDTAVYFCAKGRSSYRLNLWGQGT
LVTVSS
SEQ ID NO. 15 70H1 VL1 
DIQMTQSPSTLSASVGDRVTITCQASQSISNELSWYQQKPGKAPKLLIYRASTLESGV
PSRFSGSGSGTEFTLTISSLQPDDFATYYCQSTFYGDYGNAFGGGTKVEIK
SEQ ID NO. 16 70H1 VL2 
DIQMTQSPSTLSASVGDRVTIKCQASQSISNELSWYQQKPGKAPKLLIYRASTLE
SGVSSRFSGSGSGTEFTLTISSLQPDDFATYYCQSTFYGDYGNAFGGGTKVE IK
SEQ ID NO. 17 70H1 VL3 
DIQMTQSPSTLSASVGDRVTIKCQASQSISNELSWYQQKPGKPPKLLIYRASTLESGV
SSRFSGSGSGTEFTLTISSLQPDDFATYYCQSTFYGDYGNAFGGGTKVEIK
SEQ ID NO. 18 70H1 HCDR1 SGYDMC
SEQ ID NO. 19 70H1 HCDR2 CIYTRSSGGTYYASWAKG
SEQ ID NO. 20 70H1 HCDR3 GRSSYRLNL
SEQ ID NO. 21 70H1 LCDR1 QASQSISNELS
SEQ ID NO. 22 70H1 LCDR2 RASTLES
SEQ ID NO. 23 70H1 LCDR3 QSTFYGDYGNA
SEQ ID NO. 24 70H1 HCDR2' CIYTSSGGTYCASWAKG
SEQ ID NO. 25 70G1 HCDR1 NYAIN
SEQ ID NO. 26 70G1 HCDR2 IINGYGMTYYASWAKS
SEQ ID NO. 27 70G1 HCDR3 DRYGSYVGYSSDI
SEQ ID NO. 28 70G1 LCDR1 QASQNISSRLA
SEQ ID NO. 29 70G1 LCDR2 GTSTLAS
SEQ ID NO. 30 70G1 LCDR3 QHGYYYSSSFTTYNA
SEQ ID NO. 31 71D6 HCDR1 TSYWIY
SEQ ID NO. 32 71D6 HCDR2 CIATGSDGSTYYASWASG
SEQ ID NO. 33 71D6 HCDR3 EAGNGVYNL
SEQ ID NO. 34 71D6 LCDR1 QASESIRNALA
SEQ ID NO. 35 71D6 LCDR2 GASKLAS
SEQ ID NO. 36 71D6 LCDR3 QQYYSSSNVDNG
SEQ ID NO. 37 74F1 HCDR1 SRYNMC
SEQ ID NO. 38 74F1 HCDR2 CIYTGSSGSTYYASWAKG
SEQ ID NO. 39 74F1 HCDR3 GSYSYRLNL
SEQ ID NO. 40 74F1 LCDR1 QASQSISNELS
SEQ ID NO. 41 74F1 LCDR2 RASTLES
SEQ ID NO. 42 74F1 LCDR3 QATGYGDFGNA
SEQ ID NO. 43 69F12 HCDR1 NYDMI
SEQ ID NO. 44 69F12 HCDR2 IISSRDKAGHAAWAKR
SEQ ID NO. 45 69F12 HCDR3 RFSSMHGLDI
SEQ ID NO. 46 69F12 LCDR1 QASQSISNYLS
SEQ ID NO. 47 69F12 LCDR2 LASTLAS
SEQ ID NO. 48 69F12 LCDR3 QQGYSSDSVDNV

All documents mentioned in the present invention are incorporated herein by reference as if each document were individually incorporated by reference. Furthermore, it should be understood that after reading the foregoing teachings of the present invention, those skilled in the art can make various alterations or modifications to the present invention, and these equivalent forms also fall within the scope defined by the appended claims. .

## Claims

1. A mutant CD47 antibody, wherein the mutant CD47 antibody has one or more mutations introduced into the complementarity determining regions (CDRs) of the heavy chain variable region and/or the light chain variable region relative to the sequences of wild-type CD47 antibody, and the mutations are selected from the group consisting of:
substitution of one or more amino acids with cysteine (Cys) at positions Y33, R55, Y61, W65, Y105, or L107 of the heavy chain variable region, and/or at positions Q27, Y49, T53, F92, Y93, Y96, or N98 of the light chain variable region;
wherein, the binding affinity of the wild-type CD47 antibody for CD47 is E0, the binding affinity of the mutant CD47 antibody for CD47 is E1, and E1/E0 ≤ 5.5 (preferably E1/E0 ≤ 3, preferably ≤ 1.5, more preferably E1/E0 ≤ 1);
wherein, the wild-type CD47 antibody comprises a heavy chain variable region and a light chain variable region, and the heavy chain variable region comprises the following three heavy chain variable regions:
HCDR1 SGYDMC (SEQ ID NO. 18),
HCDR2 CIYTRSSGGTYYASWAKG (SEQ ID NO. 19),
HCDR3 GRSSYRLNL (SEQ ID NO. 20), and
the light chain variable region comprises the following three light chain variable regions:
LCDR1 QASQSISNELS (SEQ ID NO. 21),
LCDR2 RASTLES (SEQ ID NO. 22),
LCDR3 QSTFYGDYGNA (SEQ ID NO. 23);
wherein, any of the above amino acid sequences further comprises a derivative sequence optionally subjected to addition, deletion, modification and/or substitution of at least one amino acid, and capable of retaining CD47 binding affinity.

2. The mutant CD47 antibody of claim 1, wherein, the amino acid sequence of the heavy chain variable region of the mutant CD47 antibody has at least 80%, 85%, 86%, 87%, 88%, 89%, 90%, 91%, 92%, 93%, 94%, 95%, 96%, 97%, 98%, 99%, or 100% sequence identity with the amino acid sequence of the heavy chain variable region of SEQ ID NO. 7, 11, 12, 13, or 14; and
the amino acid sequence of the light chain variable region of the mutant CD47 antibody has at least 80%, 85%, 86%, 87%, 88%, 89%, 90%, 91%, 92%, 93%, 94%, 95%, 96%, 97%, 98%, 99%, or 100% sequence identity with the amino acid sequence of the light chain variable region of SEQ ID NO. 8, 15, 16, or 17.

3. The mutant CD47 antibody of claim 1, wherein, the mutant CD47 antibody comprises a modified mutant CD47 antibody having the following structure:
Ab-(P-R1)n
wherein,
Ab is a mutant CD47 antibody;
R1 is each independently a blocker;
P is a cleavable linker peptide;
n is a positive integer of 1 to 5;
"-" represents a chemical bond or a linker or a connector.

4. The mutant CD47 antibody of claim 3, wherein the blocker is selected from groups containing a polyethylene glycol-C₁₋₅ -alkylcarbonyl group: 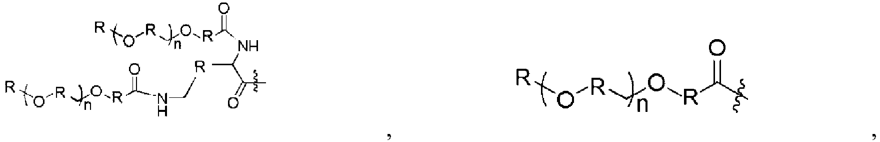 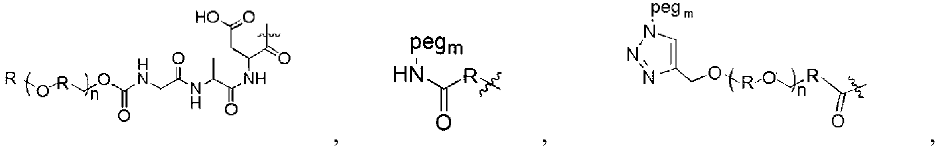 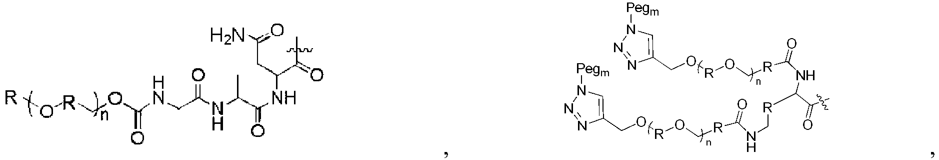 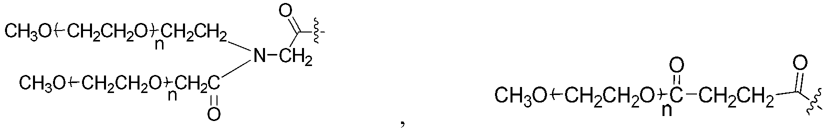 and 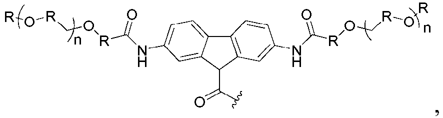 wherein each R is independently a C1-4 alkyl group; each n is independently an integer in the range of 1 to 30,000, such as an integer of 1 to 15000, 1 to 5000, 1 to 2000, 1 to 300, 1 to 150, 1 to 50, 1 to 20 or 3 to 12; polyethylene glycol or PEGₘ represents polyethylene glycol with a molecular weight of 44 to 132000, such as polyethylene glycol with a molecular weight of 1000 to 50000 or 10000 to 30000; m represents the molecular weight of the polyethylene glycol; the wavy line indicates the position connecting R2.

5. The mutant CD47 antibody of claim 3, wherein the blocker is selected from the group consisting of: PEG5K, PEG10K, PEG20K, and PEG40K.

6. An anti-CD47 antibody or an antigen-binding fragment thereof, wherein the anti-CD47 antibody comprises a heavy chain variable region and a light chain variable region,
(1) the heavy chain variable region comprises the following three heavy chain variable regions:
HCDR1 SGYDMC (SEQ ID NO. 18),
HCDR2 CIYTRSSGGTYYASWAKG (SEQ ID NO. 19),
HCDR3 GRSSYRLNL (SEQ ID NO. 20), and
the light chain variable region comprises the following three light chain variable regions:
LCDR1 QASQSISNELS (SEQ ID NO. 21),
LCDR2 RASTLES (SEQ ID NO. 22),
LCDR3 QSTFYGDYGNA (SEQ ID NO. 23); or
(2) the heavy chain variable region comprises the following three heavy chain variable regions:
HCDR1 SGYDMC (SEQ ID NO. 25),
HCDR2 CIYTRSSGGTYYASWAKG (SEQ ID NO. 26),
HCDR3 GRSSYRLNL (SEQ ID NO. 27), and
the light chain variable region comprises the following three light chain variable regions:
LCDR1 QASQSISNELS (SEQ ID NO. 28),
LCDR2 RASTLES (SEQ ID NO. 29),
LCDR3 QSTFYGDYGNA (SEQ ID NO. 30); or
(3) the heavy chain variable region comprises the following three heavy chain variable regions:
HCDR1 SGYDMC (SEQ ID NO. 31),
HCDR2 CIYTRSSGGTYYASWAKG (SEQ ID NO. 32),
HCDR3 GRSSYRLNL (SEQ ID NO. 33), and
the light chain variable region comprises the following three light chain variable regions:
LCDR1 QASQSISNELS (SEQ ID NO. 34),
LCDR2 RASTLES (SEQ ID NO. 35),
LCDR3 QSTFYGDYGNA (SEQ ID NO. 36); or
(4) the heavy chain variable region comprises the following three heavy chain variable regions:
HCDR1 SGYDMC (SEQ ID NO. 37),
HCDR2 CIYTRSSGGTYYASWAKG (SEQ ID NO. 38),
HCDR3 GRSSYRLNL (SEQ ID NO. 39), and
the light chain variable region comprises the following three light chain variable regions:
LCDR1 QASQSISNELS (SEQ ID NO. 40),
LCDR2 RASTLES (SEQ ID NO. 41),
LCDR3 QSTFYGDYGNA (SEQ ID NO. 42); or
(5) the heavy chain variable region comprises the following three heavy chain variable regions:
HCDR1 SGYDMC (SEQ ID NO. 43),
HCDR2 CIYTRSSGGTYYASWAKG (SEQ ID NO. 44),
HCDR3 GRSSYRLNL (SEQ ID NO. 45), and
the light chain variable region comprises the following three light chain variable regions:
LCDR1 QASQSISNELS (SEQ ID NO. 46),
LCDR2 RASTLES (SEQ ID NO. 47),
LCDR3 QSTFYGDYGNA (SEQ ID NO. 48);
wherein any of the above amino acid sequences further comprises a derivative sequence optionally subjected to addition, deletion, modification and/or substitution of at least one amino acid, and capable of retaining CD47 binding affinity.

7. A recombinant protein, comprising:
(1) the mutant CD47 antibody of any one of claims 1-5, or the anti-CD47 antibody or an antigen-binding fragment thereof of claim 6;
(2) an optional polypeptide molecule or fragment with therapeutic function; and/or
(3) an optional protein domain that assists in improving the physicochemical and pharmaceutical characteristics of a molecule.

8. An isolated polynucleotide encoding the mutant CD47 antibody of any one of claims 1-5, or the anti-CD47 antibody or an antigen-binding fragment thereof of claim 6.

9. A vector comprising the polynucleotide of claim 8.

10. A genetically engineered host cell, wherein the host cell comprises the vector of claim 9 or has the polynucleotide of claim 8 integrated into its genome.

11. A pharmaceutical composition, comprising:
(I) the mutant CD47 antibody of any one of claims 1-5, or the anti-CD47 antibody or an antigen-binding fragment thereof of claim 6; and
(II) a pharmaceutically acceptable carrier.

12. An immunoconjugate, comprising:
(a) the mutant CD47 antibody of any one of claims 1-5, or the anti-CD47 antibody or an antigen-binding fragment thereof of claim 6; and
(b) a coupling moiety selected from the group consisting of: a detectable label, a drug, a toxin, a cytokine, a radionuclide, an enzyme, and a combination thereof.

13. Use of the mutant CD47 antibody of any one of claims 1-5, or the anti-CD47 antibody or an antigen-binding fragment thereof of claim 6, or the immunoconjugate of claim 12, for the preparation of (a) a detection reagent or kit; and/or (b) a pharmaceutical composition for preventing and/or treating cancer or a tumor or an autoimmune disease.
